# EUROPEAN PATENT APPLICATION

(11) **EP 2 957 299 A1**
(43) Date of publication of application: **23.12.2015**
(21) Application number: 14172984.8
(22) Date of filing: 18.06.2014
(51) Int. Cl.: A61K 47/48, A61K 49/00

(54) **Peptide-based compounds and their uses for tumor imaging and targeting**

(71) Applicant: Klinikum rechts der Isar der Technischen Universität München, 81675 München (DE); Helmholtz Zentrum München Deutsches Forschungszentrum für Gesundheit und Umwelt (GmbH), 85764 Neuherberg (DE)
(72) Inventor: The designation of the inventor has not yet been filed
(74) Representative: Engelhard, Markus

(57) **Abstract**

The present invention relates to peptide-based compounds comprising (i) at least one peptide comprising the amino acid sequence of TKDNNLLGRFELXG wherein X is S or T and (ii) at least one label and/or drug. The present invention further relates to the use of said peptide-based compounds for tumor imaging and/or tumor targeting. The present invention further relates to the use of said peptide-based compounds as carrier of tumor therapeutic(s). The present invention further relates to methods for *the in vitro* and/or *in vivo* visualization, identification and/or detection of tumor cells and/or metastases as well as to methods for the treatment of cancer. The present invention further relates to a screening method for anti-tumor compounds.

## Description

The present invention relates to peptide-based compounds comprising (i) at least one peptide comprising the amino acid sequence of TKDNNLLGRFELXG wherein X is S or T and (ii) at least one label and/or drug. The present invention further relates to the use of said peptide-based compounds for tumor imaging and/or tumor targeting. The present invention further relates to the use of said peptide-based compounds as carrier of tumor therapeutic(s). The present invention further relates to methods for the *in vitro* and/or *in vivo* visualization, identification and/or detection of tumor cells and/or metastases as well as to methods for the treatment of cancer. The present invention further relates to a screening method for anti-tumor compounds.

### BACKGROUND OF THE INVENTION

Significant progress in the development of new therapies that can increase overall survival rates for a range of cancer types has been made. However, the heterogeneity within individual tumors and between tumors of the same type in different patients, as well as the different stages and sub-types of tumors, combine to confer a level of resistance to existing treatments in patients, and problems with the application of universal treatment strategies. Although chemotherapy remains one of the primary approaches for treating cancer, conventional therapy is not specifically targeted to tumor cells and is therefore associated with a high level of side-effects, some of which can be severe. The development of drug resistance and other issues associated with biodistribution and drug clearance also pose significant problems and challenges.

Although the difficulties in effectively targeting tumors that result from genetic and phenotypic heterogeneity have prompted the need for more specifically targeted approaches - 'patient stratification' and 'patient-focused medicine'. However, the ability to better treat a patient on the basis of the characteristics of their own tumor requires the identification of key target molecules or features. Although the ability to better identify tumor-associated antigens has led to the development of engineered human monoclonal antibodies (mAbs) for targeting a wide range of common malignancies (Xin et al., 2013) and the use of radiolabeled antibodies as therapeutics for different kinds of hematological malignancies and solid tumors, the production and purification of therapeutic antibodies is time-consuming and cost-intensive. Furthermore, antibodies derived from mice are not suitable for therapeutic approaches due to their immunogenicity in humans, and the affinity, avidity, or even the specificity of chimeric humanized antibodies often differ from those of the original murine antibody from which they are derived.

The primary advantage of antibodies is their specificity and ability to target single molecules bearing the epitope. However, this can also be a disadvantage, as antibodies can only target those cancers which express the antigenic determinant. It is also the case that the identification of such targets does not always result in an effective treatment. As an example, only a proportion (~50%) of those patients that bear Her2-positive breast tumors and are therefore theoretically sensitive to trastuzumab (Herceptin), respond to the drug, and many tumors develop resistance to treatment (Murphy and Morris, 2012).

Attention is now being turned towards the use of small peptides for diagnostic imaging and targeted radionuclide therapy, due to the fact that they are easy to produce with a defined and controlled purity in large quantities. Such peptides exhibit a better biodistribution compared to antibodies, are generally more stable and their enhanced ability to penetrate tissues makes them better targeting agents. Peptides can be specifically taken up via specialized receptors, and therapeutic peptides have been used in the treatment of breast (Kaumaya and Foy, 2012) and other types of cancer (Bidwell, 2012). Peptides, such as somatostatin, bombesin, cholecystokinin/gastrin, neurotensin and vasoactive intestinal peptide are currently under investigation for their possible clinical applications in nuclear oncology (Okarvi 2008). Despite the potential of these molecules, it remains essential that more universally expressed molecules which can be used as recognition structures for imaging and targeting agents are identified.

Interest in the use of peptides as innovative cancer diagnostic and tumor-specific drug delivery tools has increased (Kondo et al., 2012). However, one of the most critical issues in cancer detection using tumor-targeting probes is specificity, as most of the recently used markers also bind to tumor-associated stroma and other cells. Despite many reports on tumor markers in oncology the number of clinically useful, tumor-specific markers with prognostic relevance remains low (Schilsky and Taube, 2002; Cardoso et al., 2008).

Tumor targeting peptides represent a promising new class of prognostic tools due to their advantageous biodistribution with fast body clearance and their capacity to effectively penetrate viable cells (Kondo et al., 2012). However, it is essential that such probes exhibit specific binding to tumor cells and exclude normal tissue. The identification of tumor-selective probes is therefore essential.

Thus, there is a need in the art for improved means and methods for tumor imaging and/or targeting.

### SUMMARY OF THE INVENTION

According to the present invention this object is solved by a peptide-based compound comprising
(i) at least one peptide comprising the amino acid sequence of TKDNNLLGRFELXG [SEQ ID NO. 1] wherein X is S or T,
(ii) at least one label and/or drug.

According to the present invention this object is solved by the peptide-based compound of the present invention for use for tumor imaging and/or targeting.

According to the present invention this object is solved by the peptide-based compound of the present invention for use as carrier or vehicle of tumor therapeutic(s).

According to the present invention this object is solved by the peptide-based compound of the present invention for use for the diagnosis and/or treatment of bacterial and/or viral and/or microbial infection(s).

According to the present invention this object is solved by a method for the *in vitro* and/or *in vivo* visualization, identification and/or detection of tumor cells and/or metastases, comprising the step of administering a diagnostically amount of at least one peptide-based compound of the present invention to a patient or subject.

According to the present invention this object is solved by a method for the treatment of cancer, comprising the step of administering a therapeutically amount of at least one peptide-based compound of the present invention herein to a patient or subject in need thereof, wherein the peptide-based compound comprises tumor therapeutic(s).

According to the present invention this object is solved by a screening method for anti-tumor compounds, comprising the steps of
(a) providing a compound to be tested,
(b) providing a peptide-based compound of the present invention,
(c) providing an *in vitro* or *in vivo* tumor test system,
(d) administering or adding the compound of step (a) to the tumor test system of step (c),
(e) determining the effect of the compound to the tumor test system by administering or adding the peptide-based compound of step (b), preferably at different time points, and
   - detecting and/or visualizing tumor growth and/or tumor size over time,
   - detecting and/or visualizing metastases over time,
   - monitoring therapeutic outcome or clinical response(s) over time,
      and/or
   - screening of the at least one label and/or drug of the peptide-based compound (such as toxins, radionuclides) for personalized therapy (theranostic approaches).

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "14 to 45 amino acids" should be interpreted to include not only the explicitly recited values of 14 to 45, but also include individual values and sub-ranges within the indicated range. Thus, included in this numerical range are individual values such as 14, 15, 16, 17, 18, 19, 20,... 38, 39, 40, 41, 42, 43, 44, 45 and sub-ranges such as from 14 to 20, from 14 to 30, from 15 to 25, from 19 to 25, from 20 to 25, from 20 to 30 and from 15 to 30, etc. This same principle applies to ranges reciting only one numerical value, such as "at least 14 amino acids". Furthermore, such an interpretation should apply regardless of the breadth of the range or the characteristics being described.

### Peptide-based compounds for tumor imaging and targeting

The inventors have found that peptide-based compounds comprising a peptide comprising the amino acid sequence of TKDNNLLGRFELXG (wherein X is S or T, SEQ ID NO. 1) or consisting of said amino acid sequence that specifically bind to membrane-bound Heat shock protein 70 (Hsp70) on tumors and metastases.

Said peptide, which comprises or consists of the amino acid sequence of SEQ ID NO. 1, specifically recognizes the oligomerization domain of Hsp70 that is present on the cell surface of a broad variety of mouse and human tumors and metastases, but not on normal tissues.

Targeting membrane-bound Hsp70 on tumors offers unique advantages for detection, screening and staging of diseases_ENREF_1, as well as for drug development and treatment evaluation of a wide variety of neoplasms in theranostic approaches (Radons and Multhoff, 2005).

Heat shock protein 70 (Hsp70-1, HspA1A, #3303), the major stress-inducible member of the 70kD heat shock proteins, has been found to be selectively localized on the plasma membrane of different human tumors, representing a universal tumor-specific target on viable tumor cells with intact plasma membrane while excluding normal, healthy body cells. Screening of tumor biopsies and corresponding normal tissues of over 1,000 cases has shown that carcinoma samples of primarily diagnosed patients, but none of the tested normal tissues, frequently exhibit an Hsp70 membrane-positive phenotype_ENREF_8 (Hantschel et al., 2000). Furthermore, previously the inventors have shown that increased membrane-positivity corresponds with higher levels of cytosolic Hsp70_ENREF_9 (Nylandsted et al., 2004) in tumor cells. Most important, an Hsp70 positive tumor phenotype has been shown to be associated with a significantly decreased overall survival in cancerous diseases such as leukemia, lung, breast, lower rectal carcinomas, prostate and liver cancer. These findings demonstrate that Hsp70 membrane-positivity can serve as a useful marker of clinical outcome. Therefore, the determination of the Hsp70 tumor phenotype *in vivo* can be used for a personalized prognosis of tumor progression. To specifically target Hsp70 on the plasma membrane of viable tumor cells while excluding non-neoplastic normal cells of the tumor microenvironment, the inventors developed TPP as a peptide-based membrane Hsp70 targeting probe (peptide-based compound), comprising or consisting of amino acid sequence TKDNNLLGRFELSG (SEQ ID NO. 2) which comprises the amino acid sequence aa₄₅₀₋₄₆₃ of the C-terminal oligomerization domain of Hsp70. TPP binds to tumor cells with a high affinity *in vitro* and *in vivo.* Furthermore, the inventors observed high tumor cell-penetrating capacity of TPP after binding to membrane Hsp70, as indicated by a rapid internalization of the peptide via an endocytotic pathway. Therefore, targeting tumors with the TPP probe results in a rapid enrichment of TPP inside tumor cells. Furthermore, the highly specific binding to tumor cells with the exclusion of healthy cells and the rapid internalization results in a long resisting half-life of TPP at the tumor site while it gets efficiently washed-out from the surrounding normal tissue. TPP exhibits an improved *in vivo* biodistribution and an enhanced internalization capacity compared to an Hsp70-specific antibody termed cmHsp70.1 (see Stangl et al., 2011a; Stangl et al., 2011b). In summary, a peptide-based Hsp70 targeting offers great advantages in the specific, contrast-enhanced detection of tumors and metastases *in vivo,* and for an efficient and tumor-specific delivery of therapeutics.

As discussed above, the present invention provides a peptide-based compound comprising
(i) at least one peptide comprising or consisting of the amino acid sequence of TKDNNLLGRFELXG [SEQ ID NO. 1] wherein X is S or T,
(ii) at least one label and/or drug,

A "peptide-based compound" according to the invention refers to a compound that comprises two components, namely at least one peptide and at least one label and/or drug. The peptide-based compound can comprise further component(s).

In one embodiment, the at least one peptide comprises or consists of the amino acid sequence of TKDNNLLGRFELSG [SEQ ID NO. 2].

In one embodiment, the at least one peptide comprises or consists of the amino acid sequence of TKDNNLLGRFELTG [SEQ ID NO. 3].

The at least one label and/or drug is/are preferably covalently coupled to the peptide.

Said covalent coupling or attachment to the peptide is preferably via a linker, an amino acid side chain, to the N- and/or C-terminus (such as to both termini, C- and N-terminus).

The at least one label is preferably selected from:
- fluorescent labels,
   - such as fluorescent labels in the near infrared range for example Cy 5.5, Cy7, DyLight-750, IRDye 800CW,
   - such as fluorescent labels in the visible range for example carboxyfluorescein, FITC, PE, APC, PerCP, GFP (red and green),
- radionuclides,
   for example zirconium-89 (Zr 89), indium-111 (In111), technetium-99m (Tc99m), F-18, Cupper 64 (Cu64), gallium-68 (Ga68).
or combinations thereof.

The at least one drug is preferably selected from
- tumor (chemo)therapeutic(s),
   such as doxorubicin, ifosfamide, cyclophosphamide, taxol,
- toxin(s)
   such as amantinin, granzyme B, coffein, or combinations thereof.

In one embodiment, the peptide-based compound of the present invention comprises further component(s),
which is/are preferably selected from
- agent(s),
- tag(s),
   such as 6-His-tag, Flag-tag,
- N- and/or C-terminal modification(s),
   such as comprising acetylation and/or amidation of the *N*- and/or C-terminus,
- linker or anchoring group(s) for the label(s), drug(s) and/or agent(s),
   and/or
   combinations thereof.

The further component(s) is/are preferably covalently coupled to the peptide via a linker, an amino acid side chain, to the N- and/or C-terminus (such as to both termini, C- and N-terminus).

The peptide has preferably a length of at least 14 amino acids.

The peptide has preferably a length of 14 to about 45 amino acids, preferably 14 to about 40 amino acids or 14 to 30 amino acids, such as 14 to 25 amino acids.

In one embodiment, the peptide-based compound of the present invention comprises multiple copies of the amino acid sequence of SEQ ID NO. 1 (such as of SEQ ID NO. 2 and/or 3), such as dimers.

In one embodiment, the amino acids of SEQ ID NO. 1 (such as of SEQ ID NO. 2 and/or 3) are L- and/or D-amino acids.

A peptide-based compound of the invention has preferably at least one of the following characteristics:
(1) specific targeting and binding to the membrane bound form of Hsp70, which is exclusively present on tumor cells while absent on all normal tissue cells including cells of the tumor microenvironment;
(2) specific binding to tumor cells and metastases, but not to tumor-associated fibroblasts or macrophages,
(3) specific internalization into tumor cells but not into normal cells (advantageous for detecting tumor cells but also for delivering tumor therapeutics);
(4) selective enrichment/accumulation in tumor cells,
(5) rapid wash-out from normal tissue;
(6) specific binding to many different tumors, because membrane-Hsp70 is present at a wide variety of (human and murine) tumors;
(7) differentiation between Hsp70 positive and Hsp70 negative tumor cells within the tumor environment;
(8) high tumor-to-background contrast;
(9) strong tumor-specific signal intensity;
(10) retention time in human body only several hours;
(11) small size (preferably less than 1 kDa) compared to e.g. antibodies;
   and/or
(12) low immunogenicity, such that it can be repeatedly used for imaging and/or monitoring;

These characteristics make the peptide a versatile tool for tumor imaging and/or targeting as well as for tumor treatment.

As discussed above, the present invention provides the peptide-based compound of the present invention for use for tumor imaging and/or targeting.

As discussed above, the present invention provides the peptide-based compound of the present invention for use as carrier or vehicle of tumor therapeutic(s) or for tumor specific drug delivery.

The tumor cells and/or metastases, that can be imaged and targeted by the peptide-based compound, express heat shock protein 70 (Hsp70).

Cancers or tumors that express heat shock protein 70 (Hsp70) are:
breast cancer
prostate cancer
liver cancer
carcinomas of lung, colorectum, pancreas, larynx, stomach, peripheral and central nervous system, other carcinomas, sarcomas, chronic myeloic leukemia (CML), acute myeloic leukemia (AML), acute lymphatic leukemia (ALL), non-Hodgkin Lymphoma (NHL), myeloproliferative syndrome (MPS), myelodysplastic syndrome (MDS), plasmocytoma, other leukemias,
other malignant diseases, such as viral and/or bacterial and/or microbial infection(s), wherein Hsp70 is present on the surface of malignant or tumor cells,

In a preferred embodiment, the peptide-based compound of the present invention comprises at least one drug which is a tumor therapeutic, which is specifically carried or delivered to the tumor and/or metastases.

In a preferred embodiment, the peptide-based compound of the present invention comprises at least one label such that tumor cells and/or metastases can be specifically visualized etc.

The use according to the present invention preferably comprises the visualization, identification and/or detection of tumor cells and/or metastases, preferably *in vivo.*

Preferably, the use according to the present invention comprises
- the specific binding of the peptide-based compound to membrane-bound Hsp70, preferably the oligomerization domain of Hsp70, on tumor cells, and/or specific internalization into tumor cells;
- the specific binding and/or internalization into tumor cells and metastases but not to tumor-associated fibroblasts or macrophages.

The use according to the present invention preferably comprises diagnosis, detection, screening and/or staging of tumors.

The use according to the present invention preferably comprises therapy monitoring and/or treatment evaluation.

The use according to the present invention preferably comprises real-time tumor imaging, such as the visualization of tumor boundaries during surgery.

In one embodiment, the use according to the present invention comprises drug development and/or screening for anti-tumor compounds.

In one embodiment, the use according to the present invention comprises screening of the at least one label and/or drug of the peptide-based compound (such as toxins, radionuclides) for personalized therapy (theranostic approaches).

In one embodiment, the peptide-based agent is used as contrast agent, such as in CT or PET/CT.

In this embodiment, the peptide is preferably labeled with a radionuclide.

In one embodiment, the peptide-based compound of the present invention is used in combination with further contrast agent(s) and/or drug(s).

Preferably, the peptide-based compound is administered intravenously, subcutaneously, intraperitoneallly, intratumorally.

As discussed above, the present invention provides the peptide-based compound of the present invention for use for the diagnosis and/or treatment of viral and/or bacterial and/or microbial infection(s).

For example, plasmodium infections (e.g. malaria).

In one embodiment, the peptide-based agent is used as contrast agent, such as in CT or PET/CT.

In this embodiment, the peptide is preferably labeled with a radionuclide.

In one embodiment, the peptide-based compound of the present invention is used in combination with further contrast agent(s) and/or drug(s).

Preferably, the peptide-based compound is administered intravenously, subcutaneously, intraperitoneallly.

### Methods for tumor detection and treatment

As discussed above, the present invention provides a method for the *in vitro* and/or *in vivo* visualization, identification and/or detection of tumor cells and/or metastases.

Said method comprises the step of
administering a diagnostically amount of at least one peptide-based compound of the present invention to a patient or subject.

A "diagnostically amount" of a peptide-based compound of the present invention is preferably from about 0.01 to about 2,000 mg per kg body weight, such as 0.01 to about 1,000 mg per kg body weight.

As discussed above, the present invention provides a method for the treatment of cancer.

Said treatment method comprises the step of
administering a therapeutically amount of at least one peptide-based compound of the present invention to a patient or subject in need thereof,
wherein the peptide-based compound comprises tumor therapeutic(s).

A "therapeutically amount" of a peptide-based compound of the present invention is a similar amount as used in chemotherapies, such as preferably from about 0.01 to about 2,000 mg per kg body weight.

Preferably, the peptide-based compound is administered intravenously, subcutaneously, intraperitoneallly, intratumorally.

Preferably, the tumor cells and/or metastases express heat shock protein 70 (Hsp70), as discussed above.

Preferably, the tumors or cancers are selected from breast cancer, prostate cancer, liver cancer, carcinomas of lung, colorectum, pancreas, larynx, stomach, peripheral and central nervous system, other carcinomas, sarcomas, chronic myeloic leukemia (CML), acute myeloic leukemia (AML), acute lymphatic leukemia (ALL), non-Hodgkin Lymphoma (NHL), myeloproliferative syndrome (MPS), myelodysplastic syndrome (MDS), plasmocytoma, other leukemias,
other malignant diseases, wherein Hsp70 is present on the surface of malignant or tumor cells.

The methods of the present invention comprise preferably or are suitable for
- diagnosis, detection, screening and/or staging of tumors;
- real-time tumor imaging, such as for the visualization of tumor boundaries during surgery; and/or
- comprising therapy monitoring and/or treatment evaluation.

In one embodiment, the methods of the present invention comprise preferably or are suitable for screening of the at least one label and/or drug of the peptide-based compound (such as toxins, radionuclides) for personalized therapy (theranostic approaches).

Theranostic approaches involve preferably
- the visualization by fluorescent or radionuclide-labeled peptide-based compounds of the invention (TPP)
   and
- the treatment/therapy using toxins, drugs which are coupled to the peptide-based compounds of the invention, of a tumor/cancer,
   at the same time.

A theranostic method is a method for the *in vitro* and/or *in vivo* visualization, identification and/or detection of tumor cells and/or metastases as well as a method of treatment of cancer.

Said theranostic method according to the invention preferably comprises the steps of
(1) administering a diagnostically amount of at least one peptide-based compound of the present invention to a patient or subject, wherein the peptide-based compound comprises at least one label,
   and, preferably at the same time,
(2) administering a therapeutically amount of at least one peptide-based compound of the present invention to a patient or subject in need thereof,
   wherein the peptide-based compound comprises tumor therapeutic(s) (e.g, toxin(s), drug(s)).

The "diagnostically amount" and the "therapeutically amount" of the peptide-based compound of the present invention are as defined above.

Preferably, the peptide-based compound is administered intravenously, subcutaneously, intraperitoneallly, intratumorally.

Preferably, the tumor cells and/or metastases express heat shock protein 70 (Hsp70), as discussed above.

Preferably, the tumors or cancers are selected from breast cancer, prostate cancer, liver cancer, carcinomas of lung, colorectum, pancreas, larynx, stomach, peripheral and central nervous system, other carcinomas, sarcomas, chronic myeloic leukemia (CML), acute myeloic leukemia (AML), acute lymphatic leukemia (ALL), non-Hodgkin Lymphoma (NHL), myeloproliferative syndrome (MPS), myelodysplastic syndrome (MDS), plasmocytoma, other leukemias,
other malignant diseases, wherein Hsp70 is present on the surface of malignant or tumor cells.

### Methods for diagnosis and treatment viral, bacterial and microbial infections

As discussed above, the present invention provides a method for the diagnosis and/or treatment of viral and/or bacterial and/or microbial infections.

For example, plasmodium infections (e.g. malaria).

Said diagnosis method comprises the step of
administering a diagnostically amount of at least one peptide-based compound of the present invention to a patient or subject.

A "diagnostically amount" of a peptide-based compound of the present invention is preferably from about 0.01 to about 2,000 mg per kg body weight, such as 0.01 to about 1,000 mg per kg body weight.

Said treatment method comprises the step of
administering a therapeutically amount of at least one peptide-based compound of the present invention to a patient or subject in need thereof,
wherein the peptide-based compound comprises anti-viral and/or anti-bacterial agent(s)/drug(s).

A "therapeutically amount" of a peptide-based compound of the present invention is preferably from about 0.01 to about 2,000 mg per kg body weight.

Preferably, the peptide-based compound is administered intravenously, subcutaneously, intraperitoneallly.

The methods of the present invention comprise preferably or are suitable for
- diagnosis, detection, and/or screening staging of viral and/or bacterial and/or microbial infection(s);
   and/or
- comprising therapy monitoring and/or treatment evaluation.

### Screening methods

As discussed above, the present invention provides a screening method for anti-tumor compounds.

Said screening method comprises the steps of
(a) providing a compound to be tested,
(b) providing a peptide-based compound of the present invention,
(c) providing an *in vitro* or *in vivo* tumor test system,
(d) administering or adding the compound of step (a) to the tumor test system of step (c),
(e) determining the effect of the compound to the tumor test system by administering or adding the peptide-based compound of step (b), preferably at different time points, and
   - detecting and/or visualizing tumor growth and/or tumor size over time,
   - detecting and/or visualizing metastases over time,
   - monitoring therapeutic outcome or clinical response(s) over time,
      and/or
   - screening of the at least one label and/or drug of the peptide-based compound (such as toxins, radionuclides) for personalized therapy (theranostic approaches).

The order of steps (a) to (c) is not intended to be defined by choosing letters (a), (b) and (c). For example, their order can be:
first step (a), then step (c) and then step (b),
first step (a), then step (b) and then step (c),
or all or two of the steps (a), (b) and (c) at the same time,
   etc.

Preferably, the peptide-based compound of the present invention comprises at least one label.

Preferably, the *in vitro* or *in vivo* tumor test system is an animal model, such as a syngeneic, spontaneous or xenograft animal model, e.g. a mouse model.

In one embodiment, the *in vitro* or *in vivo* tumor test system is a cell culture test system, such as tumor cell lines of different entities.

### Further description of preferred embodiments

Herein, the inventors describe a novel peptide-based probe termed Hsp70-tumor cell penetrating peptide (TPP) that detects membrane-bound Heat shock protein 70 (Hsp70) on tumors and metastases and its use as a tool for *in vivo* tumor targeting.

TPP: TKDNNLLGRFELXG [SEQ ID NO. 1] wherein X is S or T,
TKDNNLLGRFELSG [SEQ ID NO. 2],
TKDNNLLGRFELTG [SEQ ID NO. 3].

TPP recognizes the oligomerization domain of Hsp70 that is present on the cell surface of a broad variety of mouse and human tumors and metastases, but not on normal tissues. The rapid turn-over rate of membrane-bound Hsp70 causes an accumulation of the fluorescentlylabeled TPP but not of a scrambled control peptide in malignant cells. Following intravenous (i.v.) administration the tumor homing TPP enables a site-specific labeling of tumors and metastases in syngeneic, spontaneous and xenograft mouse models. Due to its remarkable specificity for many different tumors, its internalization capacity and its rapid wash-out from normal tissues, TPP selectively enriches in tumor cells. A comparison with the commercially available non-peptide small molecule αᵥβ₃-integrin antagonist IntegriSense™ revealed a significantly higher tumor-to-background contrast and a stronger tumor-specific signal intensity of TPP in all tested tumor models. Moreover, in contrast to IntegriSense™, TPP reliably differentiates between Hsp70 positive tumor cells and Hsp70 negative cells within the tumor microenvironment, such as tumor-associated macrophages and fibroblasts.

Therefore, TPP provides a useful tool for multimodal imaging of tumors and metastases that can help to improve the understanding of tumorigenesis and is highly suitable for the diagnosis and for therapeutic monitoring. The rapid and specific internalization of TPP into tumor cells makes this tool ideal for the development of Hsp70-based targeted therapies.

For optimizing diagnostic success, one of the most critical issues in cancer targeting systems is tumor cell specificity. These requirements have been addressed by the inventors by providing the highly specific tumor cell penetrating peptide, Hsp70 TPP, targeting the membrane bound form of Hsp70, which is exclusively present on tumor cells while absent on all normal tissue cells including cells of the tumor microenvironment. Importantly, membrane-Hsp70 is present at a wide variety of human and murine tumors of different entities.

Targeting Hsp70 with small molecules, such as peptides, benefits from the rapid turnover-rate of that molecule following the endocytotic pathway (Stangl et al., 2011a), which, in consequence, leads to an accumulation of TPP in the tumor cells aligned with effectively prevented wash-out of the tissue.

In this work, the inventors demonstrate the comprehensive capacity of the novel Hsp70-targeting small molecule TTP at a variety of syngeneic and xenograft, as well as chemically induced and endogenous mouse tumor models. The use of a colitis-associated colon carcinoma model and an endogenous pancreatic tumor model best mimicked the human situation in terms of tumor morphology and tumor microenvironment, whereas xenograft carcinoma models of colon, mamma, pancreas, lung, head and neck and cervix were used to screen the performance of TPP in various human-derived carcinoma *in vivo.*

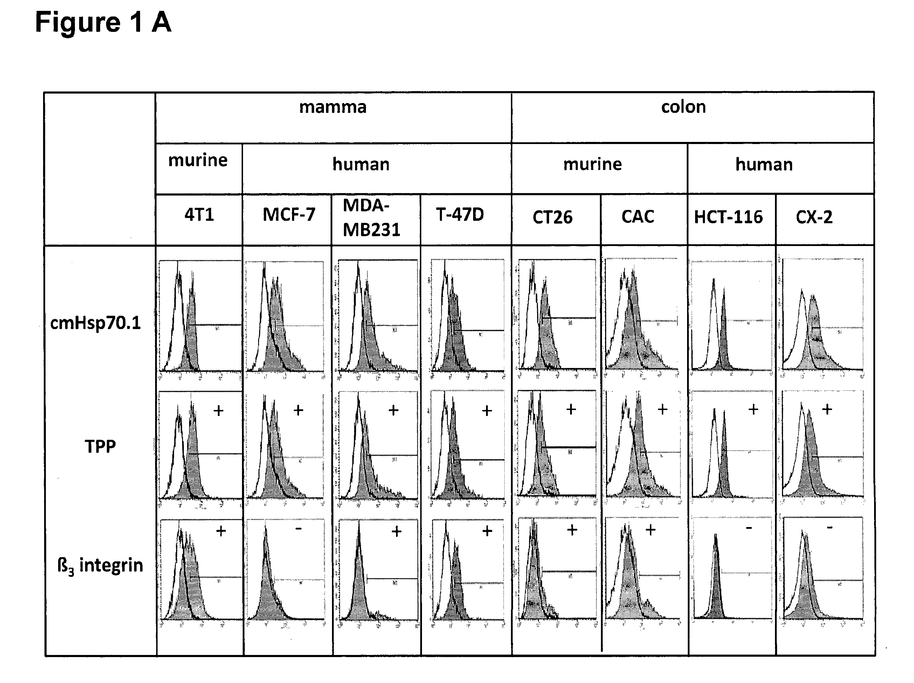

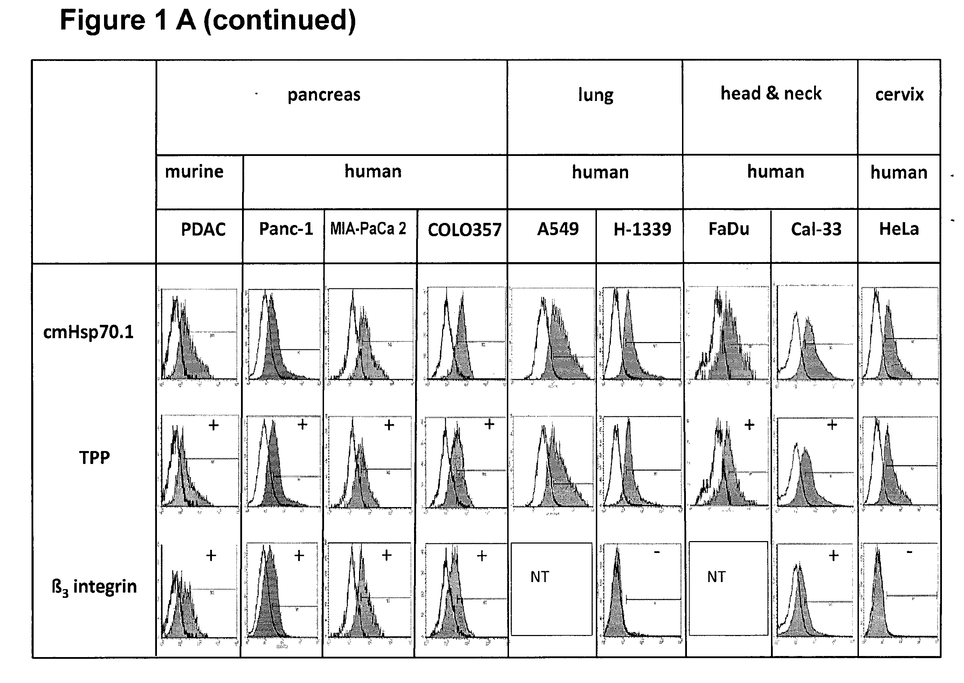

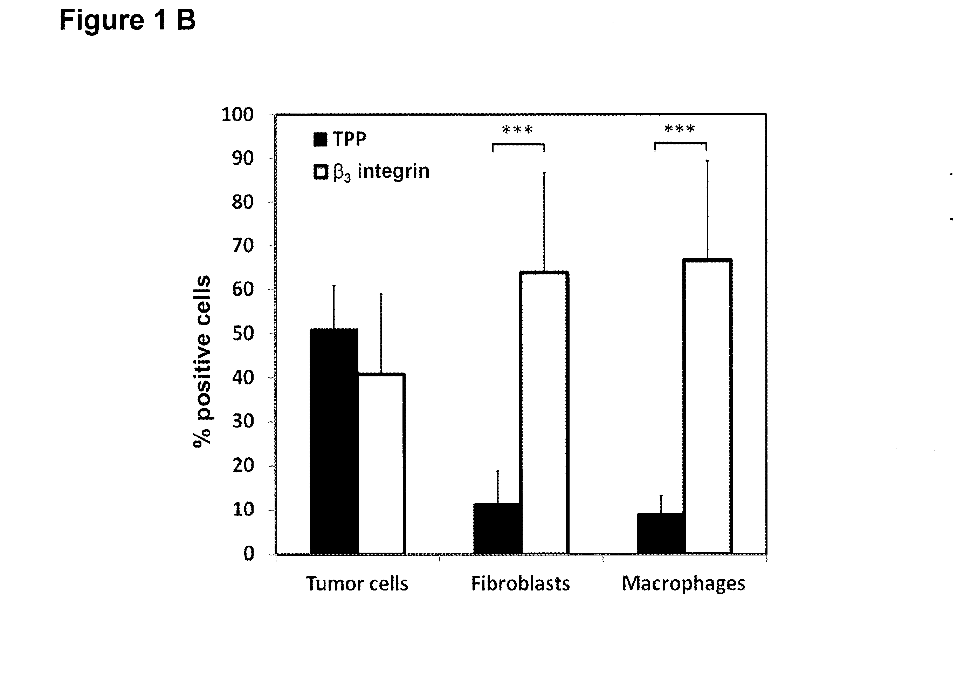

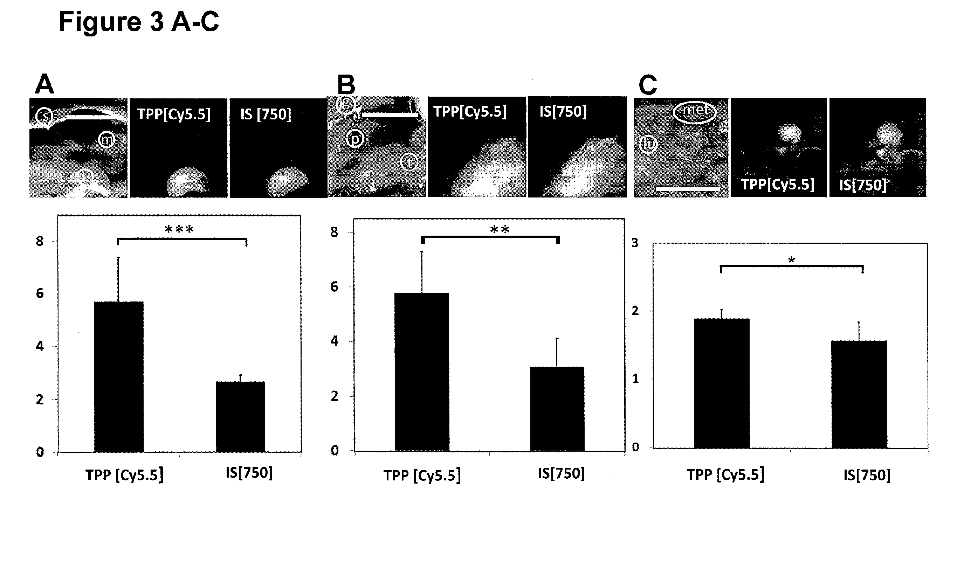

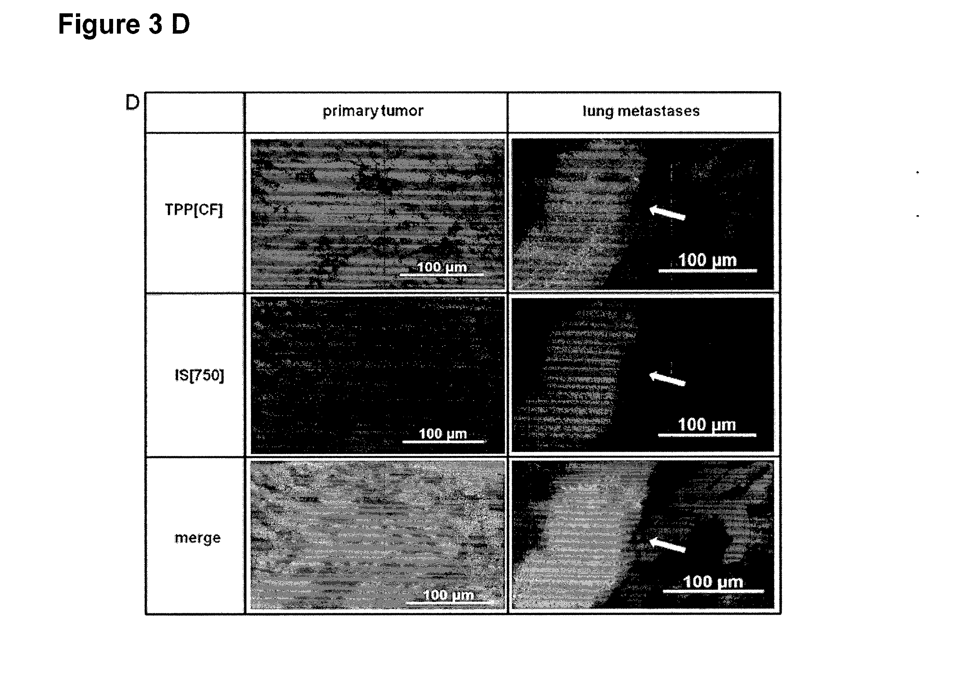

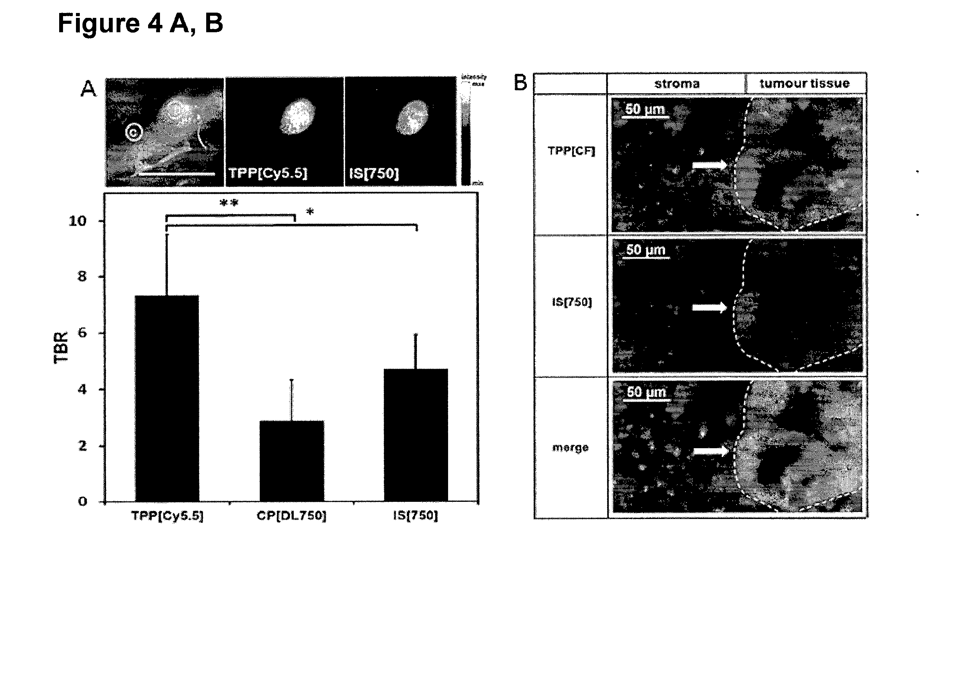

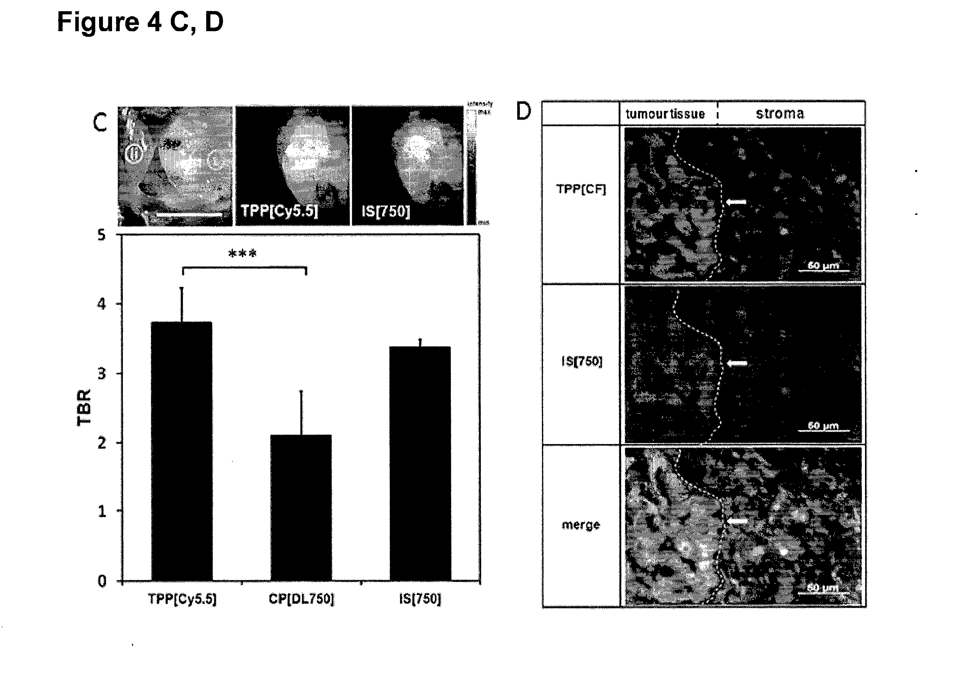

TPP was able to specifically detect and penetrate each of the tested tumors at high yields *in vivo* (see Figures 3-5, 11), as well as *in vitro* at single cell suspensions of the solid tumors (see Figure 1A and Table 1 below) with significant higher tumor to background ratios compared to a scrambled control peptide, and has further been used as the TPP molecule to be compared to the widely used tumor targeting probe IntegriSense™. For screening of the presence of the IntegriSense™ targeting epitope, β₃-integrin was used. While present at the tumor cells of the PDAC model and both tested murine cell lines (CT26, colon carcinoma and 4T1, mammary carcinoma) (see Figures 1, 4, Table 1), the IntegriSense™ target epitope was absent at 6 of the 8 human tumor cell lines tested for β₃-integrin (HCT-116, CX-2 (colon), MCF-7, MDA-MB231 (mamma), H-1339 (lung) and HeLa (cervix)), as well as at the spontaneous, colitis-associated murine colon carcinoma model.

**Table 1 Binding of TPP to different membrane-Hsp70 positive colon, mammary, pancreas, lung, head & neck and cervix mouse and human tumors. The proportion of membrane-Hsp70 positive cells was measured with cmHsp70.1 antibody and TPP peptide on single cell suspensions of in vivo-grown human and mouse tumors of different entities. The expression of CD61 (β3-integrin) was also determined on these tumor cells. A sample was determined as membrane positive if more than 20% of the cells showed a positive staining; n.t., not tested.**

| **%** | **colon** | | | | **mammary** | | | | |
|---|---|---|---|---|---|---|---|---|---|
| | **murine** | | **human** | | **murine** | **human** | | | |
| | **CT26** | **CAC** | **HCT-116** | **CX-2** | **4T1** | **MCF-7** | **MDA-MB231** | | **T-47D** |
| **Hsp7** | 55.5±5.5 | 51.2±0.8 | 63.3±9.3 | 56.6±8. | 56.3±3.5 | 58.0±3.6 | 63.3±13.3 | | 48.0±4.2 |
| **TPP** | 42.0±4.3 | 52.8±7.5 | 59.0±7.9 | 45.2±5. | 47.5±2.1 | 46.0±5.6 | 62.5±15.6 | | 43.5±2.1 |
| | 26.5±7.8 | 23.0±7.1 | 15.3±1.5 | 14.0±5. | 38.3±3.2 | 13.0±1.7 | 20.0±5.6 | | 55.5±2.1 |
| | | | | | | | | | |

| **%** | **pancreas** | | | | **lung** | | **head & neck** | | **cervix** |
|---|---|---|---|---|---|---|---|---|---|
| | **murine** | **human** | | | **human** | | **human** | | **human** |
| | **PDAC** | **Panc-1** | **MIAPa** | **COLO35** | **A549** | **H-1339** | **FaDu** | **Cal-33** | **HeLa** |
| **Hsp7** | 50.0±8 | 46.0±4 | 58.5±7 | 57.8±9. | 76.3±1 | 60.0±7 | 59.0±8 | 68.1±8 | 55.0±1 |
| **TPP** | 45.0±1 | 47.0±7 | 42.0±6 | 49.5±4. | 58.3±7 | 67.0±5 | 53.5±0 | 52.0±4 | 63.0±4. |
| **CD61** | 50.0±5 | 36.0±2 | 70.0±11. | 20.0±9. | n.t. | 4.0±1. | n.t. | 23.5±3 | 5.0±5.6 |

*In vivo* imaging of solid tumors of any tested tumor yielded higher tumor to background ratios using **TPP** compared to IntegriSense™, in β₃-integrin negative as well as β₃-integrin positive tumors. However, intraoperative *in vivo* imaging revealed remarkably high enrichment of IntegriSense™ at the tumor site. To further investigate these findings, cryo-sections of αᵥβ₃-integrin positive and -negative tumors after i.v. injection of TPP, labeled with carboxyfluorescein, in combination with IntegriSense™ 750 were analyzed. Hsp70 and integrin positive tumors showed tumor cell specific uptake of both, TPP and IntegriSense™, and additional high uptake of IntegriSense™ in tumor microenvironment, containing macrophages and fibroblasts (see Figures 3A-C). In contrast, Hsp70-positive and β₃-integrin negative tumors showed comparable high enrichment of TPP in the tumor cells. IntegriSense™ was present in the tumor microenvironment, while tumor cells remained unstained (see Figure 4). In FACS analysis of single cell suspensions of the solid tumors, we were able to detect a high membrane expression of αᵥβ₃-integrin at F4/80 positive macrophages, as well as at PDGFR-β positive fibroblasts (see Figure 1B). While *in vivo* macrophages internalized the i.v. applied TPP, there was only background staining of the peptide to tumor associated fibrous tissue detectable. Screening for the presence of Hsp70 on the plasma membrane of tumor associated macrophages as well as fibroblasts ascertained a negative result.

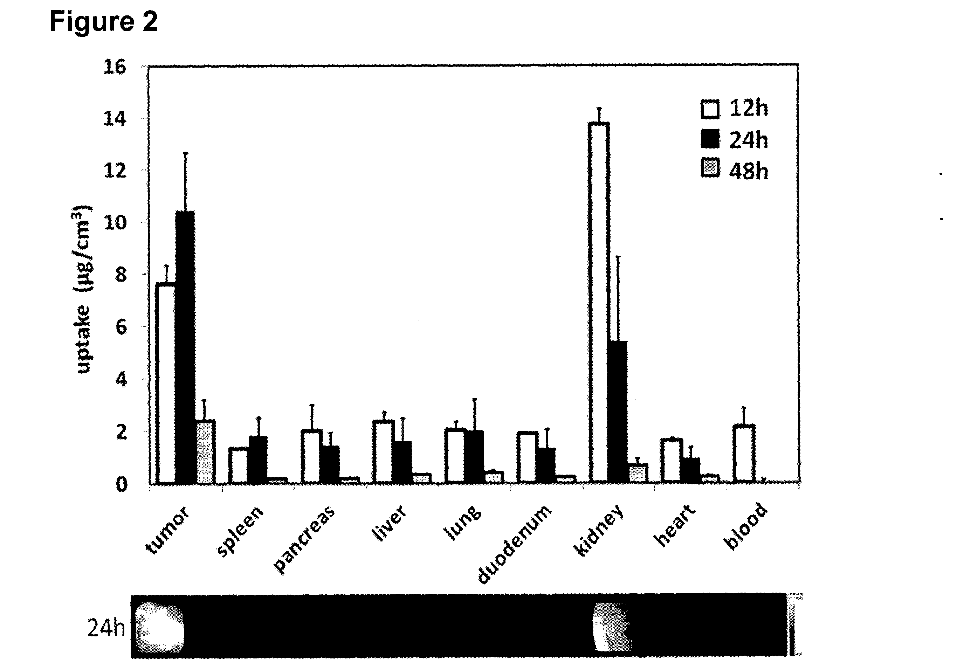

Here, the inventors could confirm the absence of Hsp70 on CD31 positive endothelial cells derived from solid tumors in previous studies_ENREF_19 (Gehrmann et al., 2012). *Ex vivo* biodistribution analysis revealed the beneficial tumor homing capacities of the peptide structure of TPP, which after 24h is effectively washed out of the normal tissue while a high proportion is still residing in the tumor tissue at this time point. As previous studies with probes of the same size indicate (Dearling et al., 2013; Huang et al., 2011), TPP is cleared out of the body via kidney while not accumulating in liver like other tumor targeting probes, as e.g. antibody like molecules. This makes TPP a potential targeting probe for hepatocellular carcinoma or liver metastases detection or their theranostic treatment (see Figure 2).

Another recently discovered tumor targeting strategy involves the chemokine receptor CXCR4. Similar to Hsp70, CXCR4 offers the possibility to target different tumor entities. However, some carcinomas, like the non-small cell lung cancer (NSCLC), do not or only weakly express CXCR4 on their plasma membranes. In contrast, Hsp70 is present on all tested NSCLC tumors (xenograft, primary human tumors) (Hantschel et al., 2000). Furthermore, potential adverse effects of targeting CXCR4, such as the mobilization of hematopoietic progenitor cells by CXCR4 antagonists, remain to be elucidated.

The current study found no evidence that TPP has any toxic side-effects, even if it was administered at 10-fold higher doses than those that were used for *in vivo* imaging. *Ex vivo* biodistribution analysis revealed the beneficial tumor-homing capacities of the TPP peptide. Although TPP was effectively washed out of the normal tissue 24 h after its administration, pronounced levels remained within the tumor tissue at this time point (Figure 2). The small molecular weight of TPP ensures that it is cleared via the kidneys and does not accumulate in the liver like high molecular weight targeting probes such as antibodies. As a consequence, TPP did not result in non-specific background signals in the liver. Therefore, TPP is a useful imaging probe for hepatocellular carcinoma and liver metastases.

In conclusion, TPP is a highly suitable peptide-based marker for diagnostic tumor targeting, as well as an efficient tool for drug delivery for a broad variety of human tumors.

The following examples and drawings illustrate the present invention without, however, limiting the same thereto.

### BRIEF DESCRIPTION OF THE DRAWINGS

**Figure 1**. *Membrane-Hsp70 and β3-integrin expression on single cell suspensions of human (h) and murine (m) tumors that were grown in mice.*
   (A) Comparison of the binding capacity of cmHsp70.1 full length antibody (top), TPP (middle) and β3-integrin antibody (lower panel) to mammary (m4T1, hMCF-7, hMDA-MB231, hT-47D), colon (mCT26, mCAC, hHCT-116, hCX-2), pancreas (mPDAC, hPanc-1, hMiaPaCa2, hCOLO357), lung (hA549, hH-1339), head & neck (hFaDu, hCal-33) and cervix (hHeLa) carcinomas. Results are expressed as log fluorescence intensity vs. relative cell numbers. The IgG1 isotype-matched control is indicated in white and membrane staining in gray histograms.
   (B) Membrane positivity of TPP (black bars) and β3-integrin (white bars) on cells of the tumor microenvironment in comparison to β3-integrin-positive tumors. Membrane staining of TPP remained significantly lower in macrophages (p=1.54x10⁻⁷) and fibroblasts (p=4.67x10⁻⁸), compared to β3-integrin. Averaged tumor cell positivity revealed no significant difference between TPP and β3-integrin staining.
**Figure 2****.** *Biodistribution of TPP[Cy5.5] in CT26 tumor-bearing mice, assessed by NIRF quantification.*
   Tumors and organs of CT26 tumor-bearing mice were collected 12, 24 and 48 h (white, black and grey bars, respectively) after i.v. injection of TPP[Cy5.5] into the tail vein. The fluorescence signals of the probe were analyzed, *ex vivo.* Two to three mice per time point were assessed; graph bars show mean values ± SD.
**Figure 3**. *Intraoperative in vivo imaging and Fluorescence microscopy of syngeneic tumor models after the injection of TPP[Cy5.5] and IS[750].* (A) NIRF imaging of CT26 tumors that were injected s.c. into the neck area, (B) 4T1 tumors injected o.t., and (C) lung metastases derived thereof.
   Upper panels, true color images of tumor (t) and metastases (met), as well as surrounding normal tissue including muscle (m), skin (s), gut (g), peritoneum (p) and lung (lu) (left, scale bars, 5 mm). Corresponding pseudocolor fluorescence images are taken 24 h after i.v. injection of TPP[Cy5.5] (middle) and IS[750] (right) into the same animal.
   Lower panel, TBR, 24 h after i.v. injection of TPP[Cy5.5] and IS[750]. The data are shown as mean ± SD (n=4 (CT26) and 6 (4T1)), * indicates p<0.05, ** indicates p<0.01 and *** indicates p<0.001.
   (D) Fluorescence microscopy of primary tumor (left) and lung tissue including metastases, indicated by arrows (right), 24 h after i.v. injection of TPP[CF] (upper panel) and IS[750]
      (middle panel) in the same animal. The lower panel displays an overlay of both compounds. Scale bars, 100 µm.
**Figure 4****.** *Intraoperative in vivo imaging and Fluorescence microscopy in spontaneous*
   *tumor mouse models 24 h after injection of TPP[Cy5.5], CP[DL750] and IS[750].*
   (A) NIRF imaging of CAC. Upper panels, true color image of tumor (t) and surrounding colon tissue (c), scale bar, 5 mm, with corresponding pseudocolor fluorescence images 24 h after i.v. injection of TPP[Cy5.5] (middle) and IS[750] (right). Lower panel, TBR, 24 h after i.v. injection of TPP[Cy5.5], CP[DL750] and IS[750]. The data are shown as mean ± SD, n=5 (TPP[Cy5.5]), 4 (CP[DL750]) and 3 (IS[750]). * indicates p<0.05 and ** indicates p<0.01.
   (B) Fluorescence microscopy of identical cryosections of a representative primary tumor (left) and normal tissue (right), 24 h after i.v. injection of TPP[CF] (upper panel) and IS[750] (middle panel) into the same animal. The lower panel displays an overlay of both compounds with enhanced enrichment of TPP[CF) in crypt-like neoplastic tissue (arrows), compared to IS[750]. Normal tissue remained negative for both compounds. Scale bars, 50 µm. (C) NIRF imaging of PDAC. Upper panel, true color image of tumor (t) and surrounding normal tissue including liver (li), (left, scale bar, 5 mm) and corresponding pseudocolor fluorescence images, 24 h after i.v. injection of TPP[Cy5.5] (middle) and IS[750] (right). Lower panels, TBR 24 h after i.v. injection of the compounds. The data are shown as mean ± SD of n=7 (TPP[Cy5.5]), 6 (CP[DL750]) and 2 (IS[750]) animals. *** indicates p<0.001.
   (D) fluorescence microscopy of identical cryosections of primary tumor tissue (left) including fibrosis of the tumor microenvironment (right), 24 h after i.v. injection of TPP[CF] (upper panel) and IS[750] (middle panel). The lower panel displays an overlay with a comparable enrichment of both compounds in carcinoma tissue and additional IS[750]-staining of cells of the tumor microenvironment. Scale bar, 50 µm.

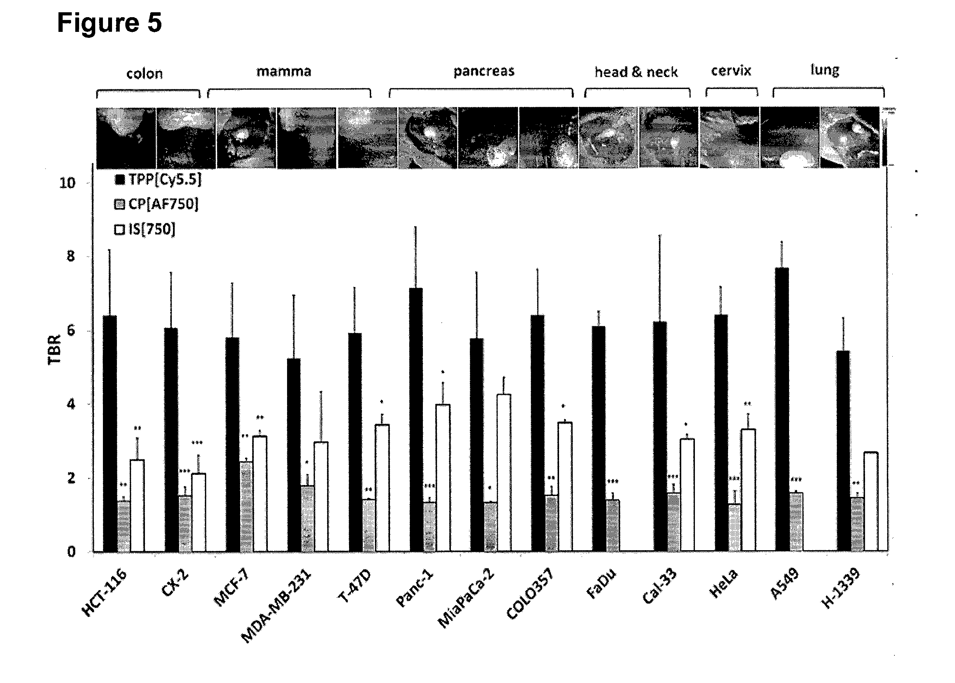

**Figure 5****.** *Intraoperative in vivo imaging of xenograft tumors, 24 h after i.v. injection of TPP[Cy5.5], CP[DL750] and IS[750].*
   Upper panel, overlay of intraoperative true color and fluorescence pseudocolor images of subcutaneous tumors and the surrounding normal tissue, 24 h after i.v. injection of TPP[Cy5.5].
   Lower panel, TBR, 24 h after i.v. injection of TPP[Cy5.5] (black bars), CP[DL750] (grey bars) and IS[750] (white bars). The data are shown as mean ± SD of 2-5 mice (H-1339, IS[750]), significance was calculated in comparison to TPP[Cy5.5] with * indicating p<0.05, ** indicates p<0.01 and *** indicating p<0.001.
Figure 6.
   (A) *Specificity of TPP to Hsp70.*
      The binding specificity of TPP peptide to Hsp70 was tested in a peptide ELISA. Hsp70 protein was coupled to 96-well ELISA plates and incubated with carboxyfluorescein labeled TPP and CP peptides at indicated concentrations (25, 50, 100 ng/ml). After an incubation period at 27°C for 30 min and two washing steps the fluorescence of carboxyfluoresceinbound peptides was measured in a multiplate ELISA reader. The data show one representative experiment out of three with similar results.
   (B) *Immunohistochemical staining of tumors using cmHsp70.1 mAb.*
      Upon immunohistochemical staining, cryosections of the PDAC model show cytosolic staining of Hsp70 in tumor cells, whereas fibroblasts of the tumor-microenvironment were negative for Hsp70. Analogous staining have been performed on tumor sections of other models with similar results. These data indicate that expression of Hsp70 only occurs in tumor cells.

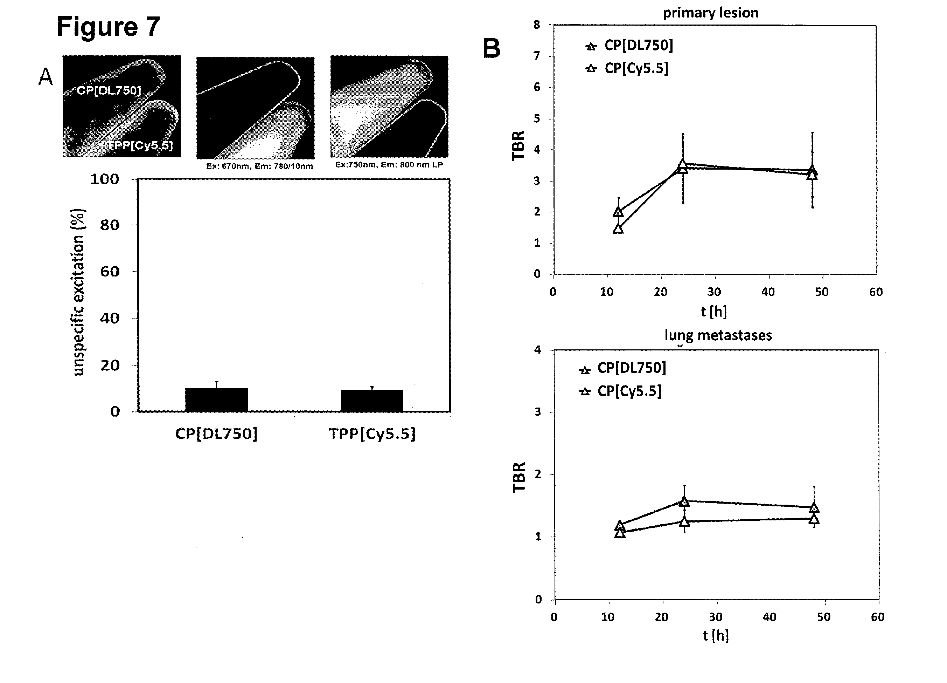

**Figure 7****.** *Discrimination of TPP[Cy.5.5] and CP[DL750] fluorescence using the NIRF imaging system.*
   Spectral unmixing of CP[DL750] and TPP[Cy5.5] was tested with two 1.5 ml centrifugation tubes, filled with 0.1 µg/ml of CP[DL750] or TPP[Cy5.5], respectively (A, upper panel). Left, color image of both tubes, middle, fluorescence signal of TPP[Cy5.5] and [CP[DL750] (right) after spectral unmixing. Quantification of unspecifically excited dye fluorescence intensities resulted in values of 9.51±1.30% for CP[DL750] and 10.17±2.76% for TPP[Cy5.5] (n=2). (B) *in vivo* confirmation of dye discrimination. 100 µg of CP[DL750] and CP[Cy5.5] were simultaneously injected i.v. into 4T1 tumor-bearing mice. Comparable tumor-to- background ratios of DyLight750 and Cy5.5 fluorescence after 12, 24 and h of circulation in primary lesions (left panel) as well as in lung metastases (right panel) confirm the comparability of the different dyes using the imaging setup and thus provides reliable signal-to-background ratios when compared *in vivo.*

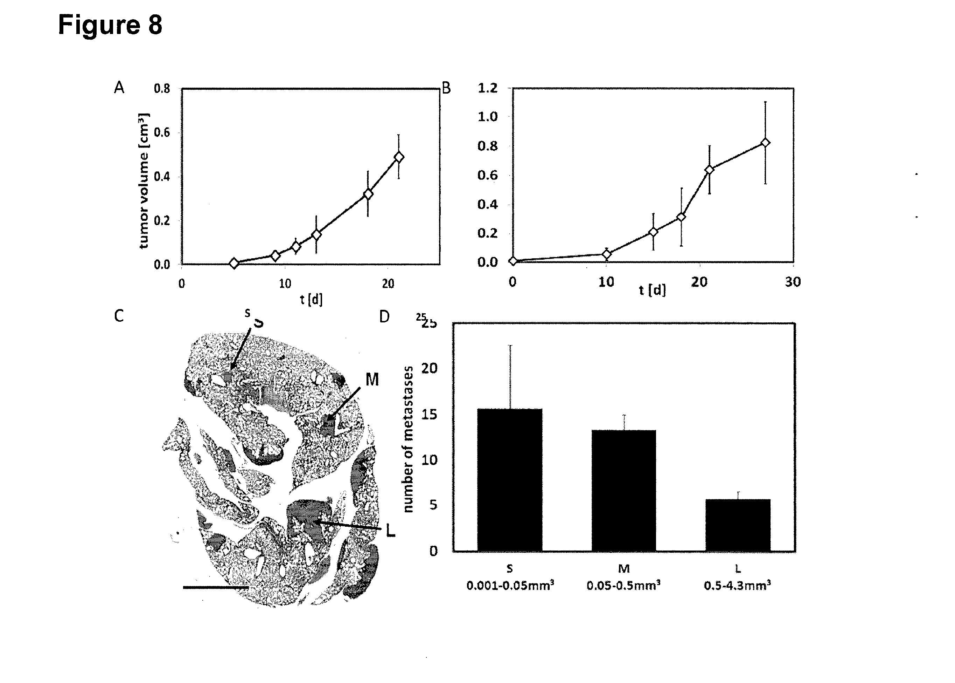

**Figure 8****.** *Primary tumor growth kinetics and size distribution of 4T1 lung metastases after s.c. CT26 and o.t. 4T1 mammary carcinoma cell implantation.*
   Primary Tumor volumes of s.c. CT26 tumors, located in the neck area (A) and o.t. 4T1 tumors (B), as determined by sonographic measurement. (C) Identification and size distribution of lung metastases after 25 d of primary tumor growth. Left, H&E stain of mouse lung containing small (S), medium-sized (M) and large (L) metastases (scale bar, 2 mm). Right, numbers of small (S, 0.001-0.05 mm³), medium-sized (M, 0.05-0.5 mm³) and large (L, 0.5-4.3 mm³) metastases of n=3 tumor-bearing mice.

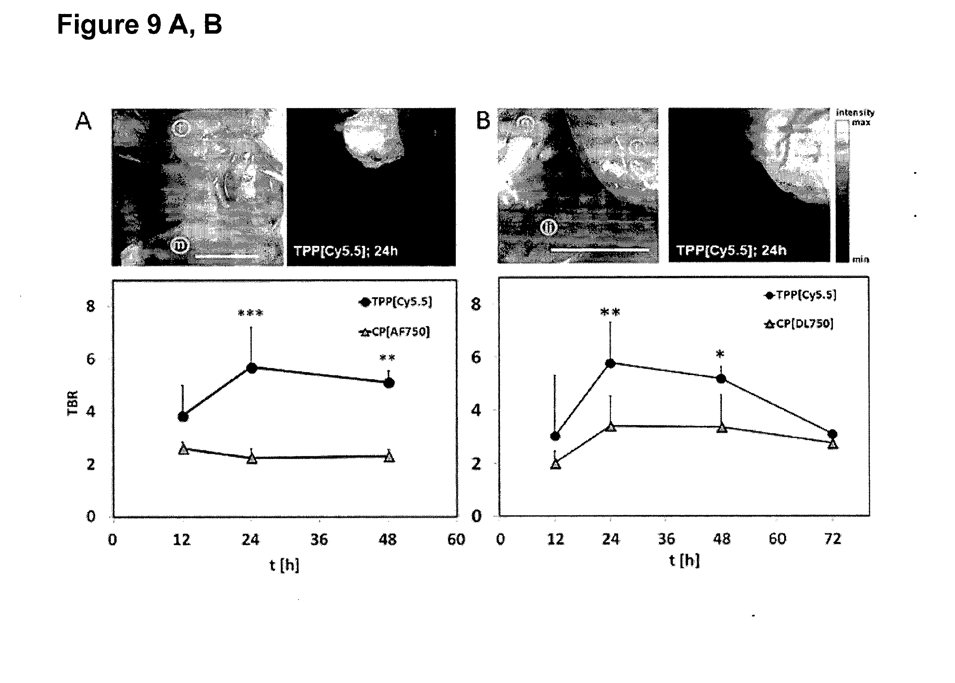

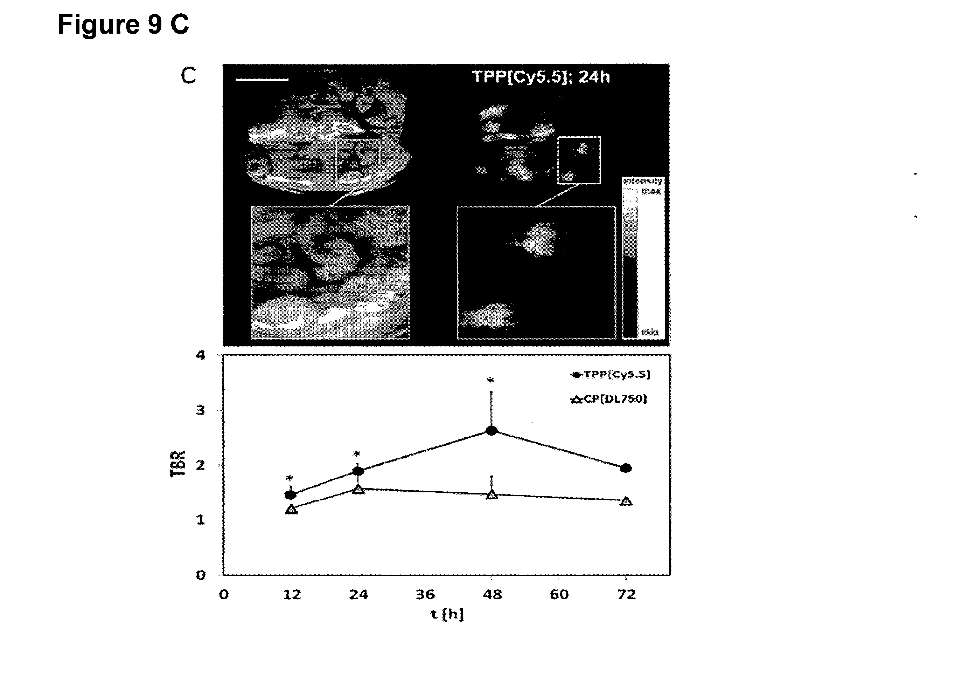

**Figure 9****.** *Time kinetics of TBR after intraoperative in vivo imaging of tumor-bearing mice after tail vein injection of TPP[Cy5*.*5*] *and CP[DL750].*
   NIRF imaging of CT26 tumors, subcutaneously implanted into the neck area (A), orthotopically implanted 4T1 tumors (B) and lung metastases derived thereof (C).
   Upper panels, true color image of tumor (t) and surrounding normal tissue including muscle (m), gut (g) and liver (li) (left) with corresponding pseudocolor fluorescence images 24 h after i.v. injection of TPP[Cy5.5] (right). Scale bars, 5 mm. Lower panels, kinetics of TBR after i.v. injection of TPP[Cy5.5] (black circles) and CP[DL750] (gray triangles), simultaneously measured in the corresponding mice. Mean signal intensities of the tumor and normal tumor-surrounding tissue were used to calculate the average tumor-to-background ratio. The TBRs indicated a progressive and tumor-specific accumulation of TPP[Cy5.5] within the first 24 h after injection of the probes in primary tumors of both models (TBR = 5.7±1.5 (CT26) and 5.8±1.5 (4T1)). The TBRs of CP[DL750] control peptide remained significant lower 24 and 48 h after probe injection. Kinetic studies of 4T1 metastases versus normal tissue ratios, calculated from epifluorescence images of dissected lungs, revealed an increase of the specific signal in tumor tissue during the first 48 h after i.v. application with a peaking ratio of 2.6±0.7 at this time point. TBRs of CP[DL750] remained significantly lower at the time-points 12, 24 and 48 h, compared to the specific probe. These data indicate that the optimal time-point for the detection of primary tumors using TPP is 24 h after i.v. application, whereas the detection of lung metastases is optimal 48 h. The data are shown as mean ± SD (n (12h) = 2 (CT26) and 4 (4T1), n (24h) = 10 (CT26) and 6 (4T1), n (48h) = 3 (CT26) and 4 (4T1), n (72h, 4T1) = 1), * indicates p<0.05, ** indicates p<0.01 and *** indicates p<0.001.

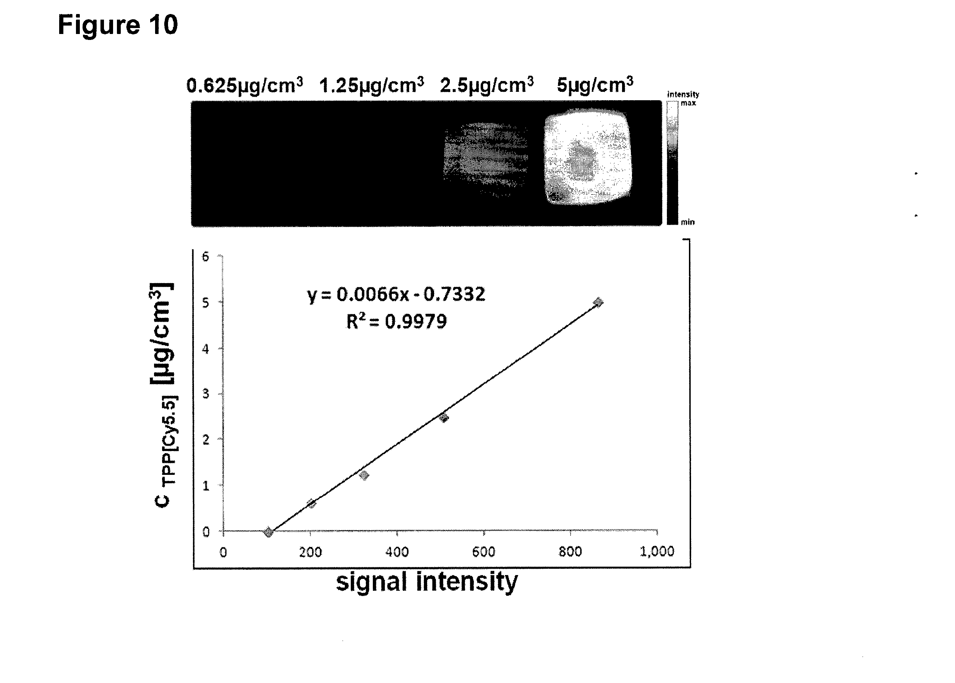

**Figure 10****.** *Repression analysis of tissue-mimicking concentration-standard gel cubes.* To quantify the contents of TPP[Cy5.5] in tumor and organs, fluorescence intensities of standardized 0.25 cm³ cubes, supplemented with 10% milk powder and containing 5, 2.5, 1.25 or 0.625 µg/cm³ TPP[Cy5.5] were measured as described in the "imaging procedures" chapter (upper panel). Regression equation of linear regression analysis (lower panel). The concentration of TPP[Cy5.5] in organs was calculated using this regression equation. Scale bar, 5 mm.

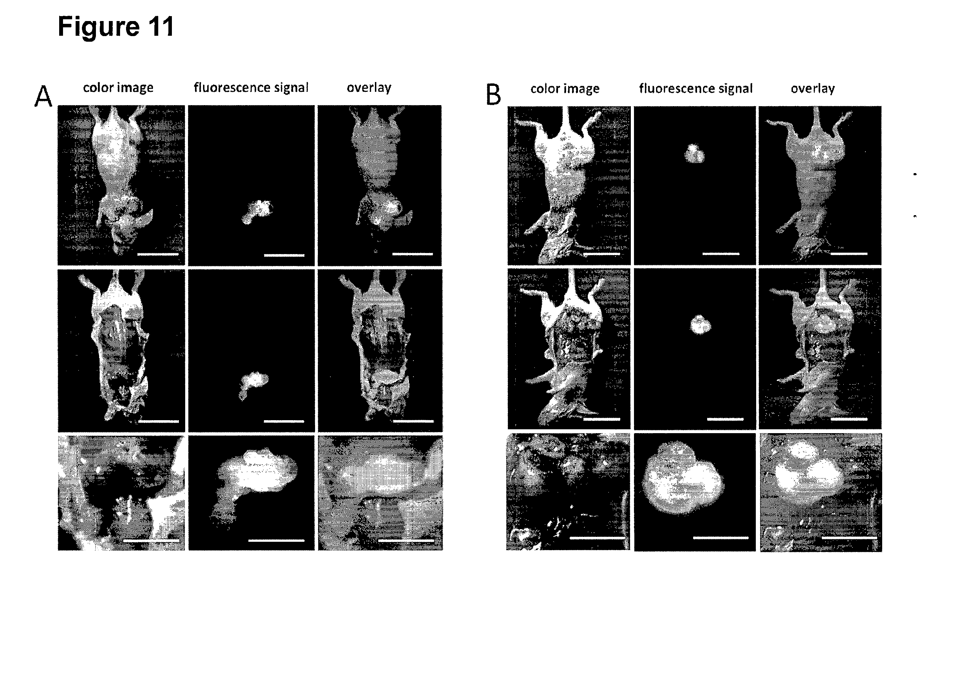

**Figure 11****.** *True color images and TPP[Cy5.5]-derived fluorescence signals of tumor-bearing mice.*
   True color images (left panels) of tumor-bearing mice after subcutaneous or orthotopical implantation of tumors, the fluorescence signals derived thereof, 24 h after i.v. injection of TPP[Cy5.5] (middle panel), as well as the overlay of color and fluorescence images (right panel).
   (A) dorsal view of a subcutaneous CT26 tumor-bearing mouse with a tumor located in the neck area.
   (B), ventral view of an orthotopic 4T1 mamma carcinoma-bearing mouse.
      Upper and middle panels: Exemplary whole-body overviews of the mice before and during intraoperative measurement (scale bar, 25 mm). Lower panels show tumor areas, captured in higher magnification in order to allow measurement of fluorescence signal intensities for calculation of TBR (scale bar, 5 mm).

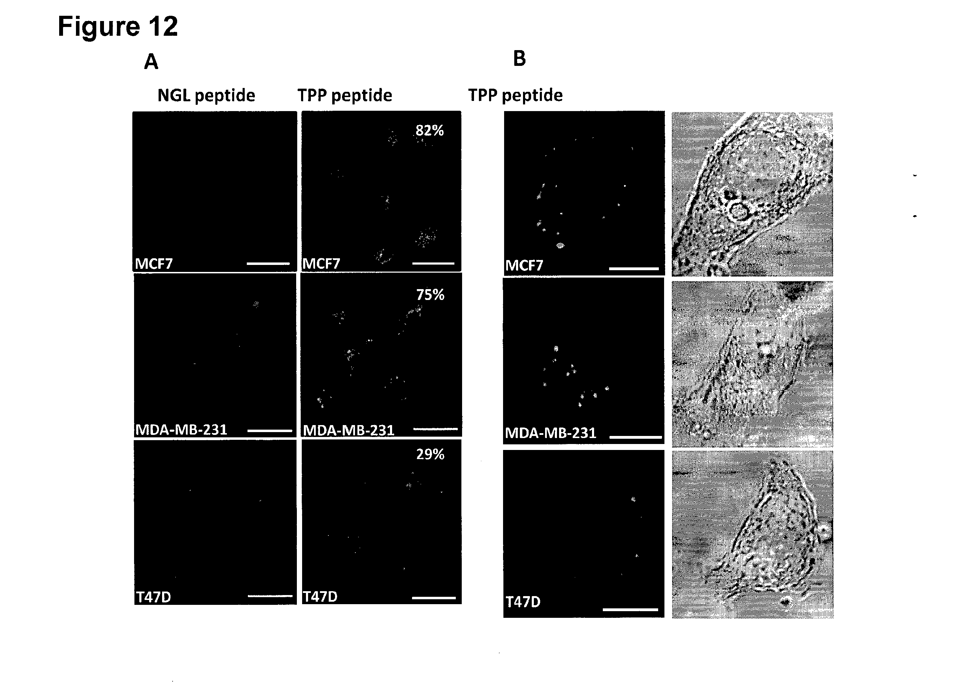

**Figure 12****.** *Specific uptake of TPP into tumor cells at 37°C* Human breast cancer cell lines (MCF7, MDA-MB-231, T47D) expressing different levels of membrane Hsp70 were incubated with CF-TPP or a scrambled peptide for 30 min at 37°C and internalization of the peptides was imaged by confocal microscopy.
   **A.** The TPP (second column, the punctuated staining reflects the localization of the TPP inside the cells) but not the scrambled control peptide NGL (left column) is internalized into the tumor cell lines. The appearance of the TPP (middle column) in well-defined, localized points suggests that the association of the peptide with intracellular vesicles is related to the membrane Hsp70 expression status. The numbers indicate the percentage of Hsp70 membrane positive stained cells. DAPI staining allows visualization of the nucleus. Objective. 20x; scale bar 50 µm
   **B.** Individual cells were imaged as a z-stack in order to better illustrate the intracellular localization of TPP. Images are representative three-frame composites from approximately mid-way in the stack. Objective 63x; scale bar 10 µm.

### EXAMPLES

### 1. Material and Methods

### 1.1 Animals

BALB/c, FvB and SHO-*Prkdc^{scid}Hr^{Hr}* (SHO) mice were obtained from an animal breeding colony (Charles River, Wilmington, MA, USA and Harlan Winkelmann, Borchen, Germany) and were maintained in pathogen-free, individually ventilated cages (Tecniplast, Hohenpeissenberg, Germany). All animal experiments were approved by the District Government of Upper Bavaria and performed in accordance with the German Animal Welfare and Ethical Guidelines of the Klinikum rechts der Isar, TUM, Munich, Germany. Tumors were examined after the animals were sacrificed by cervical dislocation.

### 1.2 Syngeneic tumor models.

CT26 (CT26.WT; ATCC CRL-2638) mouse colon adenocarcinoma cells (5x10⁵) or 4T1 (ATCC CRL-2539) mouse mammary carcinoma cells (2.5x10⁵) (American Type Culture Collection, ATCC, authentication not applicable for mouse cell lines), both derived from BALB/c mouse strains, were harvested in the exponential growth phase and injected subcutaneously (s.c.) in the neck or orthotopically (o.t.) into the mammary fad pad of 8-week-old, female BALB/c mice, respectively. Primary tumor growth was followed by ultrasonic measurements (Logiq-5, GE Healthcare, Solingen, Germany). Intraoperative *in vivo* imaging was performed post mortem on exposed tumors when the CT26 tumors reached a size of approximately 0.4 cm³. 4T1-derived tumors and lung metastases were investigated after 25 to 30 days of tumor growth.

### 1.3 Xenograft tumor models

To test the capacity of TPP to recognize human tumors, 5x10⁶ cells from the following human carcinoma cell lines were implanted subcutaneously into the neck area or orthotopically into the mammary fat pad of 8-10 week old, female SHO mice: Colon carcinoma cell lines HCT-116 (ATCC) and CX-2 (DKFZ, Heidelberg, Germany), mammary carcinoma cell lines MCF-7, MDA-MB231 and T-47D (ATCC), pancreas carcinoma cell lines Panc-1, MIA PaCa-2 (Trajkovic-Arsic et al., 2014) and COLO357 (Leibniz-Institute DSMZ, Braunschweig, Germany), small-cell lung cancer H1339, non-small cell lung cancer A549 (Schilling et al 2012), head and neck carcinoma FaDu (all ATCC) and Cal-33 (Leibniz-Institute DSMZ), as well as the human cervix carcinoma HeLa (ATCC). Mice were sacrificed and intraoperative *in vivo* imaging was performed on the exposed tumors when tumors had reached a volume of 0.2-0.4 cm³. If not stated otherwise, cell line authentication was performed by short tandem repeat profiles that are generated by simultaneous amplification of multiple short tandem repeat loci by ATCC and DSMZ. Cells were not cultured for longer than 5 months after testing.

### 1.4 Colitis-associated spontaneous tumor model (CAC)

Spontaneous colonic tumors were induced in eight-week-old female FvB mice (Charles River) using an established approach. Briefly, mice were injected intraperitoneally (i.p.) with a single dose of azoxymethane (AOM; Sigma-Aldrich, Germany) at 10 mg/kg body weight. Following AOM treatment, the mice were subjected to 3 cycles of dextran sulfate sodium salt (DSS; MP Biomedicals Germany) administration in their drinking water (cycle 1: 3%, 5 days; cycle 2: 2%, 5 days; cycle 3: 1.5%, days). After 15-18 weeks of tumor development, the mice were sacrificed and the colon was resected for experiments. The presence of colorectal tumors was confirmed using standard histological techniques.

### 1.5 Endogenous pancreatic ductal adenocarcinoma (PDAC) mouse model

The spontaneous PDAC mouse model (Ptf1a^{+/}*^{Cre};Kras*^{+/}*^{LSL-G12D} ;p53*^{*LoxP*/}*^{LoxP},* CKP) used here has been described previously (Ardito et al., 2012). After sacrificing, the abdominal cavity of tumor-bearing mice, including the pancreas, was exposed for *in vivo* imaging of the tumor area and surrounding normal tissue.

### 1.6 Toxicity testing.

Potential toxic effects of TPP was investigated by injecting healthy BALB/c mice intravenously (i.v.) with 500 and 1050 µg of TPP. Concentrations up to 500 µg of TPP per mouse were tested in tumor-bearing mice (s.c. tumors, size 0.4 cm³). The health status and the general behavior of mice were inspected. Animals were sacrificed on day 5 and the heart, liver, spleen, lung and kidneys examined for pathological changes using standard histological techniques.

### 1.7 Flow cytometry

The membrane-Hsp70 phenotype of tumors grown *in vivo* was determined by flow cytometric analysis of single cell suspensions that were generated following 30 min collagenase/dispase digestion using fluorescein-isothiocyanate (FITC)-conjugated cmHsp70.1 mAb (IgG1, multimmune GmbH, Munich) or carboxyfluorescein-conjugated TPP (TPP[CF]). Cell suspensions were incubated with the appropriate reagent for 30 min at 4°C, after which they were washed and viable (7-AAD negative) cells were analyzed using a FACSCalibur^{™} flow cytometer (Becton Dickinson, Heidelberg, Germany). Gating was applied to distinguish the CD45⁻/CD140b⁻ tumor cell population from CD45⁻/CD140b⁺ fibroblasts and CD45⁺/F4/80⁺ macrophages.

**1.8 TPP, CP, and IntegriSense™ 750 in imaging procedures.** In *in vivo* imaging experiments, Cy5.5 or DyLight750-conjugated Hsp70 specific 14-mer tumor penetrating peptide (TPP, OEM manufactured by Thermo Fischer, Ulm, Germany) was compared with the unspecific scrambled control peptide (CP, OEM manufactured by Thermo Fischer, Ulm, Germany) and with IntegriSense™ 750 (IS750, PerkinElmer, MA, USA) (Figure 7).

| | | |
|---|---|---|
| TPP | TKDNNLLGRFELSG | SEQ ID NO. 2 |
| scrambled control peptide (CP) | NGLTLKNDFSRLEG | SEQ ID NO. 4 |

The intra-operative imaging procedure which captured color and fluorescence images of the exposed tumors and its surrounding normal tissue was configured to simulate an intraoperative imaging situation. TPP[Cy5.5] or CP[DL750] (100 µg, equal to 45 nmol per animal) or IS750, at the recommended dose of 2 nmol per animal, were injected into the tail vein of tumor-bearing mice. Concentrations above 2 nmol resulted in a higher staining of healthy tissues and thus negatively impacted the tumor-to-background ratio (data not shown). Signal specificity was determined by calculating the ratio of the mean signal intensity in the exposed tumor tissue and that of the surrounding normal tissue (tumor-to-background ratio, TBR) (Themelis et al., 2011). Kinetic studies were performed 12, 24, 48 and 72 h after tail vein bolus injection of TPP[Cy5.5] and CP[DL750]. All other experiments were performed 24 h after i.v. injection of the respective probes.

Fluorescent images were acquired by illuminating the specimens using 670 nm and 740 nm diode lasers and guiding the emitted fluorescence through appropriate emission filters before capturing it using a back illuminated EM-CCD camera (iXon DU888, Andor, Belfast, Northern Ireland), as described previously (Stangl et al, 2011 a).

A more detailed description of the imaging procedures:
In order to acquire fluorescent images during selective illumination of the specimen with white light or diode lasers with wavelengths of 670 nm or 750 nm a back illuminated EM-CCD camera was used. For visual survey of the tumor borders and a superior ability to align the fluorescence signal with the tumor tissue, as well as to enhance the precision of fluorescence signal quantification, we performed intraoperative *in vivo* imaging experiments on exposed tumors. Light from the tissue was collected using a fixed focal length objective lens optimized for infrared transmission (Planar 1.4/50 ZF-IR, Carl Zeiss, Oberkochen, Germany). The light collected by the objective was filtered using a 780/10 nm band pass filter for Cy5.5 dye and a 800 nm long pass filter for DyLight750 or IntegriSense 750. 670 nm and 750 nm CW diode lasers (B&W Tek, Newark, DE, USA) at 300 mW were selectively used for the excitation of Cy5.5 and DyLight750 or IntegriSense 750, respectively. The laser light beam was guided through a multimode fiber (200 µm core/ 0.22 NA) to a collimator and a diffuser (F260SMA-b, ED1-S20, Thorlabs, Newton, NJ, USA) for beam expansion and uniform illumination. For color images, light was filtered by red, green and blue dichroic color filters. A 250 W halogen lamp (KL-2500 LCD, Edmund Optics) was used for white light illumination. To measure fluorescence of the Cy5.5 and DyLight750 / IntegriSense-750 signals, exposure times of 0.2, 0.5, 1 and 2 s were applied using the 780/10 nm band pass filter / 670 nm laser combination or the 800 nm LP filter/750 nm laser combination, respectively. In all experiments, the specimen was excited with laser sources adjusted to a current of 0.7 mA.

The selection of the regions for tumor and background was done by two independent researchers in a blinded way. Within the field of view (1.2 x 1.2 cm²) the complete tumor and normal tissue areas were gated macroscopically as separate regions of interest (ROI) in the true color image mode. ROI were then transferred one-to-one to the fluorescence images. Mean fluorescence intensities of the respective ROIs using unprocessed images were determined by using image J 1.48v software (NIH, USA) to calculate the tumor-to background ratios. A verification of the different tissue types (tumor and surrounding normal tissue) was done by a histological inspection of representative sections of both ROIs by a pathologist.

A single injection of the control peptide CP or TPP coupled to either DL750 (CP[DL750] or Cy5.5 (TPP[Cy5.5] did not result in different tumor-to-background ratios when compared to mice that were co-injected with both peptides CP[DL750] and TPP[Cy5.5] simultaneously. A comparison of the unspecific excitation of both coupled peptides (CP[DL750] and TPP[Cy5.5] showed no significant differences in primary lesions and lung metastases of mice as indicated in Figure 8A and B.

### 1.8 Biodistribution studies

For biodistribution tumor-bearing mice were sacrificed 12, 24 or 48 h after i.v. injection of TPP[Cy5.5]. Fluorescent signal intensities of 0.25 cm³ tissue cubes, taken from the tumor, spleen, pancreas, liver, lung, duodenum, kidney, heart, and 0.25 ml of peripheral blood were measured as described above. Agarose-gel cubes with standardized concentrations were used to calculate the probe concentrations in the investigated tissue samples. Detailed information concerning the quantification of TPP[Cy5.5] is provided in Figure 10.

After the mice had been sacrificed, the contents of TPP[Cy5.5] in the tumors and organs (spleen, pancreas, liver, lung, duodenum, kidney, heart and blood) were assessed. For this, standardized 0.25 cm³ cubes of the tissues were cut using a 0.25 x 0.25cm x 0.25 cm form cutter with an open end and a scalpel to cut at this defined height. Biodistribution measurements were performed at time points of 12, 24 and 48 h of circulation in the body. Fluorescence intensities of the tissues were measured as described in the imaging procedures chapter. Quantification of the Cy5.5 contents in the defined tissue volumes was calculated using fluorescence signal intensities of standards with defined concentrations. To generate these standards, 5, 2.5, 1.25 and 0.625 µg/ml TPP[Cy5.5] were dissolved in 1% agarose gel cubes, supplemented with 10% milk powder to mimic the optical properties of body tissue, and cut in the manner described above. Fluorescence signal intensity was measured using the same instrument settings than for the tissue specimen (Figure 10, upper panel). A control-cube containing 10% milk-powder in 1% agarose did not emit any fluorescence in the Cy5.5 channel. Linear regression equation of the standards was calculated with Microsoft Excel 2007 (Figure 10, lower panel). The concentration of TPP[Cy5.5] in organs was calculated using the regression equation.

**1.9 Histology, immunohistochemistry (IHC) and fluorescence microscopy**

Paraformaldehyde-fixed and paraffin-embedded tissue sections (2 µm) were stained with hematoxylin and eosin (H&E) using standard procedures. For IHC on cryosections, unspecific mouse-on-mouse reactions were blocked using a M.O.M. kit (Vector Labs, Burlingame, CA) and stained with cmHsp70.1 mAb following antigen retrieval in citrate buffer. The quantification and volumetric analysis of lung metastases was performed using consecutive tissue sections (100 µm) using Aperio ImageScope software (Leica, Nussloch, Germany).

For microscopic *in situ* fluorescence analysis, TPP[CF] (100 µg) and the recommended dose of IntegriSense™ 750 (IS[750]) was injected i.v. into tumor-bearing mice and allowed to circulate for 24 h. Tissue was collected and 8 µm cryosections counterstained with DAPI (DAKO, Hamburg, Germany) and examined microscopically using a Zeiss Observer Z1 (Carl Zeiss, Oberkochen, Germany) equipped with standard filters for GFP and Cy7 after 4 min of fixation with 1.8% w/v PFA.

### 1.10 Statistics

Statistical analysis was performed using Student's t-test. Data are mean ± SD. P values ≤ 0.05 were considered as being significant different between the compared groups.

### 2. Results

### 2.1 The membrane-bound form of Hsp70, but not β3-integrin, is present on a wide variety of human and murine tumors.

To examine the presence of the investigated reporter epitopes on cells *in vivo,* a broad variety of murine and human tumors were screened for the presence of membrane-Hsp70 and β3-integrin (CD61), the target epitopes for TPP and IntegriSense™, respectively. This screening included colon, mammary, pancreatic, lung, head and neck, and cervical carcinomas derived from syngeneic, chemically-induced, spontaneous and xenograft mouse models. Flow cytometric analysis of viable cells derived from the solid tumors demonstrated that all of the cells examined exhibited a comparable membrane-Hsp70 staining pattern using the cmHsp70.1 monoclonal antibody or the TPP probe (Figure 1, Table 1). This finding further emphasizes the fact that targeting membrane-Hsp70 could serve as a universal approach for diagnosing a wide variety of tumor entities.

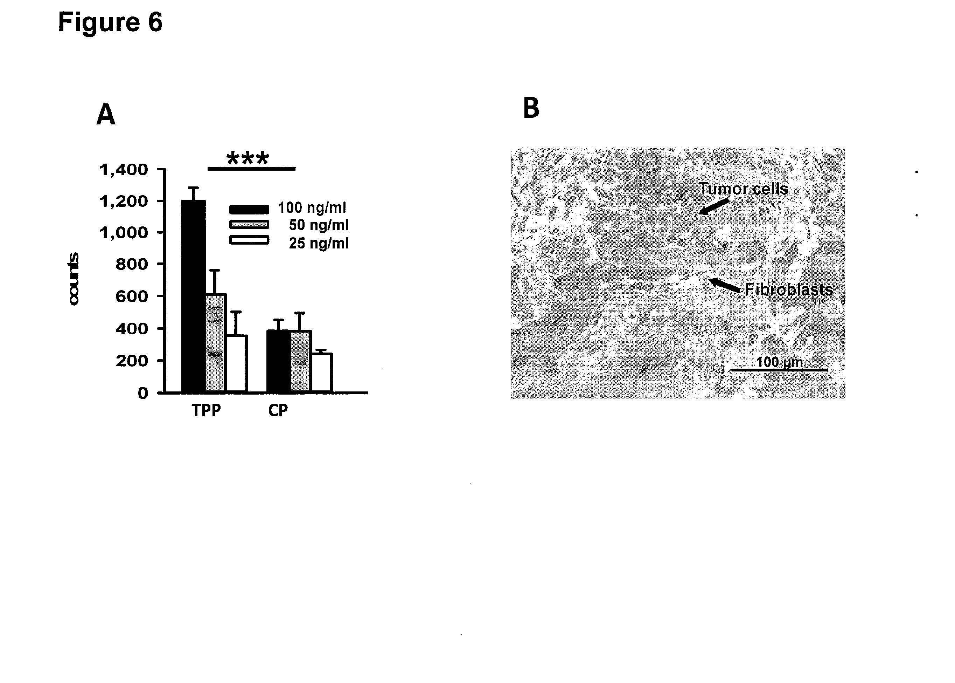

For flow cytometric analysis, a cell population was defined to be membrane-positive if as more than 20% of the cells were stained. The threshold of 20% was defined upon screening of a large panel of healthy human tissues including human peripheral blood lymphocytes, fibroblast, and healthy tissues surrounding tumor (n=86). CD61 staining revealed a β₃-integrin positivity on mouse colon (CT26) and mammary (4T1) carcinoma, on human mammary (MDA-MB231, T-47D) and head and neck (CAL-33) carcinoma, as well as on human and mouse pancreatic carcinoma (PDAC, Panc-1, MIA PaCa-2, COLO357). However, no β₃-integrin expression could be detected on human colon (HCT-116, CX-2), mammary (MCF-7), lung (H-1339) or cervical (HeLa) carcinoma cells. Cells derived from a murine colitis associated carcinoma (CAC) model showed a heterogeneous CD61 expression. These data indicate that, in comparison to β₃-integrin, Hsp70 is more frequently expressed on human and murine tumors. However, tumor cell specificity is critical for targeting strategies. Therefore, we investigated the presence of membrane-Hsp70 and β3-integrin on tumor-residing CD45⁺/F4/80⁺ macrophages and CD45⁻/CD140b⁺ fibroblasts was investigated. TPP only weakly stains (below 10%) tumor-associated fibroblasts and tumor-infiltrating macrophages; in contrast, β3-integrin positivity was found on more than 60% of tumor-associated fibroblasts and tumor-infiltrating macrophages (Figure 1B). To test Hsp70 positivity on tumors, cryosections were stained with the Hsp70 specific antibody cmHsp70.1. Whereas tumor cells of the various tumor models were positive, cells of the tumor microenvironment remained negative for Hsp70. A representative staining of a PDAC tumor is shown in Figure 6B.

### 2.2 TPP is not toxic in vivo.

To examine potential toxic side effects of TPP, healthy and tumor-bearing BALB/c mice were injected i.v. with 500 to 1050 µg of TPP, which is equivalent to 25 to 50 µg/g of their body weight. Gross monitoring of the mice did not show any toxic reactions such as skin ulcerations or toxic death at any of the tested doses. Nor did any animal exhibit any loss of weight or change in mobility. Histological examination of the heart, liver, spleen, lung and kidney on day 5 after injection by a veterinarian, revealed no pathological changes. An examination of the effects of TPP concentrations up to 500 µg in tumor-bearing mice also did not exert any side effects.

### 2.3 TPP accumulates within the tumor with maximal specificity 24 h post i.v. injection.

To investigate the *in vivo* circulation and tumor-homing properties of TPP, we analyzed its biodistribution in CT26 tumor-bearing mice. The accumulation of TPP in different organs was determined 12, 24 and 48 h after i.v. injection of 100 µg TPP[Cy5.5] by measuring fluorescent signal intensities in the tumor, spleen, pancreas, liver, lung, duodenum, kidney, heart, and in the peripheral blood (Figure 2). A strong TPP[Cy5.5] signal could be detected within the tumor (7.6±0.7 µg/cm³) 12 h after i.v. injection. This reached a maximum after 24 h (10.4±2.2 µg/cm³) and dropped after 48 h. TPP accumulation was significantly lower at all investigated time points in all other tested organs, except the kidney. The concentration of TPP[Cy5.5] reached its maximum in the kidney after 12 h at 13.7±0.6 µg/cm³, and this accumulation was followed by a sharp drop, 24 and 48 h post injection. These data further confirm the tumor-specificity of TPP and indicate that TPP is cleared from the body by renal excretion.

In further experiments, we investigated the optimal time point *for in vivo* imaging in a CT26 tumor model (s.c.) 2 weeks after tumor injection (Figure 9A) and a metastasizing 4T1 mammary carcinoma model (o.t.) 3-4 weeks after tumor injection (Figure 9B-C, Figure 11). TPP[Cy5.5] yielded maximal tumor-to-background ratio (TBR) in primary tumors 24 h after injection of the probe, whereas the peak TBR in metastasized lungs occurred after 48 h. The scrambled control peptide CP[DL750] did not show any specific accumulation in tumor tissue (Figure 9A-C).

### 2.4. The specific accumulation of TPP in syngeneic primary tumors and metastases is superior to that of IntegriSense™.

To confirm the specificity of TPP[Cy5.5] for primary tumors and metastases *in vivo,* we simultaneously injected 100 µg of TPP[Cy5.5] and the recommended dose of IntegriSense™ 750 (IS[750]) into CT26 and 4T1 tumor-bearing mice, 24 h prior to *in vivo* imaging. Although TPP[Cy5.5] and IS[750] both clearly delineate the tumor, IS[750] signals can also be found in normal tissue surrounding the tumor (Figure 3A-B, true color images of the region of interest is depicted on the left). TPP[Cy5.5] exhibited a significantly higher TBR than IS[750] (p=0.0004 and 0.003, respectively).

To further investigate the specificity of the probes, *ex vivo* imaging of dissected lungs containing metastases was performed. Quantification of metastases can be found in Figure 8. The TBR for IS[750] imaging was also lower than that for TPP[Cy5.5] (p=0.04; Figure 3C, lower panel) in distant lesions. Although the delineation of metastases by TPP[Cy5.5] and IS[750] was comparable, the enrichment of IS[750] in normal lung tissue is marginally higher (Figure 3C, upper panel).

The specificity and co-localization of TPP and IS was further confirmed on cryo-sections of the lesions which were obtained 24 h after i.v. injection of TPP[CF] and IS[750] (Figure 3D). Both probes specifically enriched in the primary tumor and lung metastases, however, a background-staining of IS[750] remained in healthy lung tissue. These data demonstrate that, although both compounds specifically stain tumors and metastases, TPP is more effectively washed out of normal tissue and therefore provides a better signal-to-background contrast than IntegriSense™.

### 2.5 The specific tumor-homing capacity of TPP is higher than that of IntegriSense™ in endogenous tumor models.

To more realistically mimic the histological and molecular features of human tumors in *in vivo* imaging, we took advantage of chemically-induced and endogenous mouse models of colorectal (CAC) and pancreatic (PDAC) cancer. Flow cytometric analysis of isolated tumor cells revealed Hsp70 and β3-integrin positivity in both models (see Table 1).

To investigate the *in vivo* tumor targeting properties in these models, NIR imaging of tumors was performed 24 h after i.v. injection of TPP[Cy5.5], CP[DL750] and IS[750] (Figure 4). Although the fluorescence signals of both TPP[Cy5.5] and IS[750] clearly delineated the exposed colon and pancreatic tumors, a clear background staining in the tumor-surrounding normal colon tissue was observed following administration of IS[750]. In the colitis-induced tumors, the TBR of TPP[Cy5.5] was significantly higher than that of CP[DL750] (p=0.005) and IS[750] (p=0.016) (Figure 4A). The cellular distribution of the compounds at a microscopic level was analyzed using cryosections of the resected tumors that were obtained 24 h after i.v. injection of TPP[CF] and IS[750]. Although a strong cytosolic signal of TPP[CF] in predominantly crypt-like tumor structures (white arrows) was observed, IS[750] treatment resulted in a weaker and more heterogeneous staining pattern (Figure 4B).

We could also show that membrane-Hsp70 is rapidly endocytosed in tumor cells under physiological conditions which provides an explanation for the cytosolic staining pattern of tumors. See Figure 12 which shows the uptake of TPP in three tumor (mamma carcinoma) cell lines which differ in their Hsp70 membrane expression (as shown in Figure 1), wherein TPP internalizes best in tumor cells with high Hsp70 membrane expression (Figure 3A, right column) whereas scrambled control peptide (NGL) is not internalized (Figure 3A, left column).

| | | |
|---|---|---|
| scrambled control peptide (NGL) | NGLTLKNDFSRLEG | SEQ ID NO. 4 |

The uptake capacities of TPP and IntegriSense™ were also investigated using an endogenous pancreatic PDAC model which has an activating mutation in the *Kras* oncogene concomitant with a deletion of the tumor suppressor *Tp53* (Ardito et al., 2012). Tumor cells of this model exhibited strong membrane positivity of both Hsp70 and β3-integrin (seeTable 1). Imaging of the pancreatic tumors was performed after opening of the peritoneal cavity; the stomach, liver and gut were used to calculate the background fluorescence. The TBR for TPP[Cy5.5] was significantly higher than that of the control peptide (p=0.0001) and comparable to that of the integrin αᵥβ₃-targeting compound IS[750] (Figure 4C). Figure 4D shows fluorescence images of a cryo-sliced pancreatic tumors 24 h after simultaneous injection of TPP[CF] and IS[750]. Although both compounds stained tumor cells equally (left, white arrows), cells of the tumor microenvironment showed a weaker staining with TPP[CF] (right).

### 2.6 TPP has a higher capacity to specifically target human xenograft tumors than IntegriSense™.

To confirm the tumor homing capacities of TPP[Cy5.5] in human tumors, imaging experiments were performed in 13 different human tumor cell lines derived from 6 entities (colon, mammary, pancreas, head and neck, cervix and lung; Figure 5). The TBRs for TPP[Cy5.5] in the xenograft tumors ranged from 5.6±1.6 (mammary carcinoma cell line, MDA-MB231) to 7.8±1.4 (squamous cell carcinoma cell line of the head and neck, Cal-33), all of which were significantly higher than those of CP[DL750]. This indicates a specific enrichment of TPP[Cy5.5] in all of the investigated xenograft tumors. Since it is known that the majority of human pancreatic carcinomas are positive for β₃-integrin (Themelis et al., 2011), we included 3 pancreas tumor xenograft models (Panc-1, MiaPaCa-2, COLO357) in our experiment. These models revealed substantial Hsp70 and β₃-integrin positivity (Table 1, Figure 1). Although IS[750] produced higher TBRs in the pancreatic tumors compared to the other xenograft models that were examined, TPP[Cy5.5] yielded significantly higher ratios in two of these three models (p=0.04 (Panc-1) and p=0.03 (COLO357)).

Other human carcinoma cell lines which were determined as being β3-integrin positive were T-47D, MDA-MB-231 (mammary carcinomas) and Cal-33 (squamous cell carcinoma of the head and neck; see Table 1). The TBRs of IS[750] fluorescence of T-47D and Cal-33 also were significantly lower than the TBRs for TPP[Cy5.5] (p=0.04 and 0.02, respectively). The TBR for IS[750] in all β3-integrin negative tumors remained clearly below the TBRs for TPP[750].

These data indicate that TPP is a powerful tumor targeting agent for the detection, diagnosis and targeting of several entities in humans.

The features disclosed in the foregoing description, in the claims and/or in the accompanying drawings may, both separately and in any combination thereof, be material for realizing the invention in diverse forms thereof.

### REFERENCES

Ardito, C. M. et al. EGF receptor is required for KRAS-induced pancreatic tumorigenesis. Cancer cell 22, 304-317, doi:10.1016/j.ccr.2012.07.024 (2012).
Bidwell GL. Peptides for cancer therapy: a drug-development opportunity and a drug-delivery challenge. Ther Deliv. 2012 May;3(5):609-21.
Cardoso F, Saghatchian M, Thompson A, Rutgers E, Committee TCS. Inconsistent criteria used in American Society of Clinical Oncology 2007 update of recommendations for the use of tumor markers in breast cancer. J Clin Oncol 2008;26:2058-9.
Dearling JL, Barnes JW, Panigrahy D, Zimmerman RE, Fahey F, Treves ST, Morrison MS, Kieran MW, Packard AB. Specific uptake of 99mTc-NC100692, an αvβ3-targeted imaging probe, in subcutaneous and orthotopic tumors. Nucl Med Biol. 40(6):788-94. (2013).
Eckmann, L. et al. Opposing functions of IKKbeta during acute and chronic intestinal inflammation. Proceedings of the National Academy of Sciences of the United States of America 105, 15058-15063, doi:10.1073/pnas.0808216105 (2008).
Gehrmann, M. et al. Immunotherapeutic targeting of membrane Hsp70-expressing tumors using recombinant human granzyme B. PloS one 7, e41341, doi:10.1371/journal.pone.0041341 (2012).
Hantschel, M. et al. Hsp70 plasma membrane expression on primary tumor biopsy material and bone marrow of leukemic patients. Cell Stress.Chaperones. 5, 438-442 (2000).
Hosotani, R. et al. Expression of integrin alphaVbeta3 in pancreatic carcinoma: relation to MMP-2 activation and lymph node metastasis. Pancreas 25, e30-35 (2002).
Huang CW, Li Z, Conti PS. In vivo near-infrared fluorescence imaging of integrin α2β1 in prostate cancer with cell-penetrating-peptide-conjugated DGEA probe. J Nucl Med. 52(12):1979-86. (2011).
Kaumaya PT, Foy KC. Peptide vaccines and targeting HER and VEGF proteins may offer a potentially new paradigm in cancer immunotherapy. Future Oncol. 2012 Aug;8(8):961-87.
Kondo E, Saito K, Tashiro Y, Kamide K, Uno S, Furuya T, et al. Tumour lineage-homing cell-penetrating peptides as anticancer molecular delivery systems. Nat Com 2012;3:951.
Murphy CG, Morris PG. Recent advances in novel targeted therapies for HER2-positive breast cancer. Anticancer Drugs 2012 Sep;23(8):765-76.
Nylandsted, J. et al. Heat shock protein 70 promotes cell survival by inhibiting lysosomal membrane permeabilization. The Journal of experimental medicine 200, 425-435, doi:10.1084/jem.20040531 (2004).
Okarvi SM. Peptide-based radiopharmaceuticals and cytotoxic conjugates: potential tools against cancer. Cancel Treat Rev. 2008 Feb;34(1):13-26.
Schilling D, Bayer C, Li W, Molls M, Vaupel P, Multhoff G. Radiosensitization of normoxic and hypoxic h1339 lung tumor cells by heat shock protein 90 inhibition is independent of hypoxia inducible factor-1α. PLoS One. 2012;7(2): e31110. Epub 2012 Feb 7.
Schilsky RL, Taube SE. Tumor markers as clinical cancer tests - are we there yet? Sem Oncol 2002;29:211-2.
Stangl, S. et al. In vivo imaging of CT26 mouse tumours by using cmHsp70.1 monoclonal antibody. Journal of cellular and molecular medicine 15, 874-887, doi:10.1111/j.1582-4934.2010.01067.x (2011).
Stangl, S. et al. Targeting membrane heat-shock protein 70 (Hsp70) on tumors by cmHsp70.1 antibody. Proceeding of the National Academy of Sciences of the United States of America 108, 733-738, doi:10.1073/pnas.1016065108 (2011).
Themelis G, Harlaar NJ, Kelder W, Bart J, Sarantopoulos A, van Dam GM, et al. Enhancing surgical vision by using real-time imaging of alphaVbeta3-integrin targeted near-infrared fluorescent agent. Annals Surg Oncol 2011;18:3506-13.
Trajkovic-Arsic M, Mohajerani P, Sarantopoulos A, Kalideris E, Steiger K, Esposito I, Ma X, Themelis G, Burton N, Michalski CW, Kleeff J, Stangl S, Beer AJ, Pohle K, Wester HJ, Schmid RM, Braren R, Ntziachristos V, Siveke JT. Multimodal molecular imaging of integrin αvβ3 for in vivo detection of pancreatic cancer. J Nucl Med. 55(3):446-51 (2014).
Xin L, Cao J, Cheng H, Zeng F, Hu X et al. (2013) Human monoclonal antibodies in cancer therapy: a review of recent developments. Front Biosci 18: 765-772.

## Claims

1. A peptide-based compound comprising
(i) at least one peptide comprising the amino acid sequence of TKDNNLLGRFELXG [SEQ ID NO. 1] wherein X is S or T,
(ii) at least one label and/or drug, preferably covalently coupled to the peptide.

2. The peptide-based compound of claim 1, wherein the at least one label is selected from:
- fluorescent labels,
- such as fluorescent labels in the near infrared range
for example Cy 5.5, Cy7, DyLight-750, IRDye 800CW,
- such as fluorescent labels in the visible range
for example carboxyfluorescein, FITC, PE, APC, PerCP, GFP (red and green),
- radionuclides,
for example zirconium-89 (Zr 89), indium-111 (In111), technetium-99m (Tc99m), F-18, Cupper 64 (Cu64), gallium-68 (Ga68).
or combinations thereof,
and/or wherein the at least one drug is selected from tumor (chemo)therapeutic(s) (such as doxorubicin, ifosfamide, cyclophosphamide, taxol), toxin(s) (such as amantinin, granzyme B, coffein) or combinations thereof.

3. The peptide-based compound of claim 1 or 2, comprising further component(s), which is/are preferably selected from
- agent(s),
- tag(s),
such as 6-His-tag, Flag-tag,
- N- and/or C-terminal modification(s),
such as comprising acetylation and/or amidation of the N- and/or C-terminus,
- linker or anchoring group(s) for the label(s), drug(s) and/or agent(s),
and/or
combinations thereof.

4. The peptide-based compound of any of claims 1 to 3, wherein the at least one label and/or drug and/or the further component(s) is/are covalently coupled to the peptide via a linker, an amino acid side chain, to the N- and/or C-terminus,
and/or wherein the peptide has a length of 14 to 45 amino acids, preferably 14 to 30 amino acids,
and/or wherein the peptide comprises multiple copies of the amino acid sequence of SEQ ID NO. 1.

5. The peptide-based compound of any of claims 1 to 4 for use
for tumor imaging and/or targeting
and/or as carrier of tumor therapeutic(s).

6. The peptide-based compound for use according to claim 5 comprising the visualization, identification and/or detection of tumor cells and/or metastases, preferably *in vivo,*
wherein the tumor cells and/or metastases express heat shock protein 70 (Hsp70),
wherein the tumors are preferably selected from breast cancer, prostate cancer, liver cancer, carcinomas of lung, colorectum, pancreas, larynx, stomach, peripheral and central nervous system, other carcinomas, sarcomas, chronic myeloic leukemia (CML), acute myeloic leukemia (AML), acute lymphatic leukemia (ALL), non-Hodgkin Lymphoma (NHL), myeloproliferative syndrome (MPS), myelodysplastic syndrome (MDS), plasmocytoma, other leukemias,
other malignant diseases, wherein Hsp70 is present on the surface of malignant or tumor cells.

7. The peptide-based compound for use according to any of claims 5 or 6, comprising
- the specific binding of the peptide-based compound to membrane-bound Hsp70, preferably the oligomerization domain of Hsp70, on tumor cells, and/or specific internalization into tumor cells;
- the specific binding and/or internalization into tumor cells and metastases, but not to tumor-associated fibroblasts or macrophages.

8. The peptide-based compound for use according to any of claims 5 to 7, comprising
- diagnosis, detection, screening and/or staging of tumors;
- therapy monitoring and/or treatment evaluation;
- real-time tumor imaging, such as for the visualization of tumor boundaries during surgery;
- drug development and screening for anti-tumor compounds.
and/or
- screening the at least one label and/or drug (such as toxins, radionuclides) for personalized therapy (theranostic approaches).

9. The peptide-based compound of any of claims 1 to 4 for use for the diagnosis and/or treatment of viral and/or bacterial and/or microbial infection(s), such as malaria.

10. The peptide-based compound for use according to any of claims 5 to 9, wherein the peptide-based agent is used as contrast agent,
and/or wherein the peptide-based compound is used in combination with further contrast agent(s) and/or drug(s),
and/or wherein the peptide-based compound is administered intravenously, subcutaneously, intraperitoneallly, intratumorally.

11. A method for the *in vitro* and/or *in vivo* visualization, identification and/or detection of tumor cells and/or metastases, comprising the step of
administering a diagnostically amount of at least one peptide-based compound of any of claims 1 to 4 to a patient or subject.

12. A method for the treatment of cancer, comprising the step of
administering a therapeutically amount of at least one peptide-based compound of any of claims 1 to 4 to a patient or subject in need thereof,
wherein the peptide-based compound comprises tumor therapeutic(s).

13. The method of claim 11 or 12, wherein the peptide-based compound is administered intravenously, subcutaneously, intraperitoneallly, intratumorally,
and/or wherein the tumor cells and/or metastases express heat shock protein 70 (Hsp70),
wherein the tumors are preferably selected from breast cancer, prostate cancer, liver cancer, carcinomas of lung, colorectum, pancreas, larynx, stomach, peripheral and central nervous system, other carcinomas, sarcomas, chronic myeloic leukemia (CML), acute myeloic leukemia (AML), acute lymphatic leukemia (ALL), non-Hodgkin Lymphoma (NHL), myeloproliferative syndrome (MPS), myelodysplastic syndrome (MDS), plasmocytoma, other leukemias,
other malignant diseases, wherein Hsp70 is present on the surface of malignant or tumor cells.

14. The method of any of claims 11 to 13, comprising
- diagnosis, detection, screening and/or staging of tumors;
- real-time tumor imaging, such as for the visualization of tumor boundaries during surgery;
- therapy monitoring and/or treatment evaluation;
and/or
- screening the at least one label and/or drug (such as toxins, radionuclides) for personalized therapy (theranostic approaches).

15. A screening method for anti-tumor compounds,
comprising the steps of
(a) providing a compound to be tested,
(b) providing a peptide-based compound of any of claims 1 to 4,
(c) providing an *in vitro* or *in vivo* tumor test system,
(d) administering or adding the compound of step (a) to the tumor test system of step (c),
(e) determining the effect of the compound to the tumor test system by administering or adding the peptide-based compound of step (b), preferably at different time points, and
- detecting and/or visualizing tumor growth and/or tumor size over time,
- detecting and/or visualizing metastases over time
- monitoring therapeutic outcome or clinical response(s) over time. and/or
- screening of the at least one label and/or drug of the peptide-based compound (such as toxins, radionuclides) for personalized therapy (theranostic approaches),
wherein preferably the *in vitro* or *in vivo* tumor test system is an animal model (such as a syngeneic, spontaneous or xenograft animal model, e.g. a mouse model) or a cell culture test system.


SEQUENCE LISTING

<110>  Klinikum rechts der Isar der TU München

<120>  Peptide-based compounds and their uses for tumor imaging and
       targeting

<130>  U30521EP

<160>  4

<170>  PatentIn version 3.5

<210>  1
<211>  14
<212>  PRT
<213>  Artificial Sequence

<220>
<223>  TPP peptide wherein Xaa is Ser or Thr


<220>
<221>  misc_feature
<222>  (13)..(13)
<223>  Xaa can be any naturally occurring amino acid

<400>  1

Thr Lys Asp Asn Asn Leu Leu Gly Arg Phe Glu Leu Xaa Gly
1               5                   10


<210>  2
<211>  14
<212>  PRT
<213>  Homo sapiens

<400>  2

Thr Lys Asp Asn Asn Leu Leu Gly Arg Phe Glu Leu Ser Gly
1               5                   10


<210>  3
<211>  14
<212>  PRT
<213>  Homo sapiens

<400>  3

Thr Lys Asp Asn Asn Leu Leu Gly Arg Phe Glu Leu Thr Gly
1               5                   10


<210>  4
<211>  14
<212>  PRT
<213>  Artificial Sequence

<220>
<223>  scrambled control peptide

<400>  4

Asn Gly Leu Thr Leu Lys Asn Asp Phe Ser Arg Leu Glu Gly
1               5                   10